# EUROPÄISCHE PATENTSCHRIFT

(11) **EP 0 732 330 B1**
(45) Veröffentlichungstag und Bekanntmachung des Hinweises auf die Patenterteilung: **12.09.2001**
(21) Anmeldenummer: 96103434.5
(22) Anmeldetag: 06.03.1996
(51) Int. Cl.: C07D 319/06, C09K 19/34

(54) **Di-1,3-Dioxan-Derivate als Flüssigkristalle**
Di-1,3-dioxane derivatives useful as liquid crystals
Dérivés di-1,3-dioxane comme cristaux-liquides

(30) Priorität: 16.03.1995 CH 75495
(43) Veröffentlichungstag der Anmeldung: 18.09.1996
(73) Patentinhaber: Rolic AG, 6301 Zug (CH)
(72) Erfinder: Buchecker, Richard, 8008 Zürich (CH); Marck, Guy, 68170 Rixheim (FR); Villiger, Alois, 4056 Basel (CH)
(74) Vertreter: Groner, Manfred

(56) Entgegenhaltungen:
- EP-A- 0 087 679
- EP-A- 0 433 836
- WO-A-91/11497
- GB-A- 2 105 717
- PATENT ABSTRACTS OF JAPAN vol. 18, no. 668 (C-1289), 16.Dezember 1994 & JP-A-06 263711 (DAINIPPON INK & CHEM. INC.), 20.September 1994,
- 'Kirk-Othmer Encyclopedia of Chemical Technology 3rd edition volume 14 page 401', JOHN WILEY & SON
- 'Landolt-Bornstein,New series,Group IV Macroscopic properties of matter, Volume 7, Liquid Crystals',
- G.W.GREY: "Liquid Crystals page 56", 1987, JOHN WILEY & SON,

## Beschreibung

Die vorliegende Erfindung betrifft neuartige flüssigkristalline Verbindungen mit zwei 1,3-Dioxanringen, deren Dipolmomente gleichgerichtet sind. Die Erfindung betrifft ebenfalls flüssigkristalline Mischungen enthaltend solche Di-Dioxan-Derivate, sowie die Verwendung solcher Verbindungen und Mischungen in elektro-optischen Vorrichtungen.

Flüssige Kristalle werden vor allem als Dielektrika in Anzeigevorrichtungen verwendet, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super twisted nematic"), SBE-Zellen ("super birefringence effect") und OMI-Zellen ("optical mode interference"). Für Displays mit hohem Informationsgehalt sind in letzter Zeit neben den passiv angesteuerten, multiplexierten Zellen, besonders die aktiv angesteuerten, z.B. TFT-Zellen ("thin film transistor"), wichtig geworden. Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische, photochemische und thermische Stabilität und eine gute Stabilität gegenüber elektrischen Feldern besitzen. Ferner sollten sie über einen möglichst breiten Bereich eine geeignete Mesophase aufweisen (beispielsweise für die Kj/5.12.95 oben genannten Zellen eine nematische bzw. eine cholesterische Phase), sie sollten aber trotzdem eine ausreichend niedere Viskosität besitzen, um in den Zellen kurze Ansprechzeiten, tiefe Schwellenspannungen und einen hohen Kontrast zu ermöglichen. Weitere Eigenschaften, wie die elektrische Leitfähigkeit, die dielektrische Anisotropie und die optische Anisotropie, müssen je nach Anwendungsgebiet und Zellentyp unterschiedlichen Anforderungen genügen. Beispielsweise sollten Materialien für die Zellen mit verdrillt nematischer Struktur eine möglichst hohe positive dielektrische Anisotropie und gleichzeitig eine möglichst geringe Leitfähigkeit aufweisen. Die letztere Eigenschaft ist vor allem für aktiv adressierte Flüssigkristallanzeigen mit hoher Informationsdichte, z.B. TFT-Zellen, von besonderer Wichtigkeit. Leider führen aber Komponenten mit höherer dielektrischer Anisotropie infolge ihres besseren Lösungsvermögens für ionische Verunreinigungen meist zu einer erhöhten Leitfähigkeit in Mischungen. Es werden daher Komponenten gesucht, die sich durch eine möglichst hohe dielektrische Anisotropie bei gleichzeitig möglichst geringer Leitfähigkeit auszeichnen. Flüssigkristalline Verbindungen, unter anderem Dioxan Derivate, mit einer hohen positiven dielektrischen Anisotropie sind beispielsweise in JP-A-6°263°711 beschrieben.

Es wurde gefunden, dass durch den Einbau eines neuartigen Bausteines, nämlich einer Di-Dioxan-Gruppe anstelle der üblichen Ringkombinationen, flüssigkristalline Verbindungen erhalten werden, welche sich durch eine sehr hohe dielektrische Anisotropie (Δε) und eine ausserordentlich niedrige Leitfähigkeit auszeichnen. Diese Verbindungen sind zudem überraschend stabil und eignen sich auch bezüglich der anderen Eigenschaften hervorragend als Komponenten für Flüssigkristallgemische.

Die vorliegende Erfindung betrifft somit Verbindungen der allgemeinen Formel 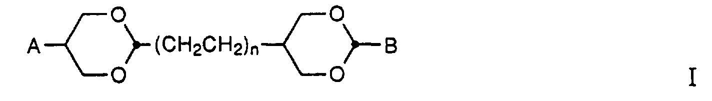 worin
- A und B: unabhängig voneinander cyclische oder acyclische hydrophobe Reste bedeuten; und
- n: 0, 1 oder 2 ist.

Die erfindungsgemässen flüssigkristallinen Verbindungen weisen eine ausserordentlich niedrige optische Anisotropie (Δn) auf. Gleichzeitig ist, wie bereits erwähnt, ihre dielektrische Anisotropie (Δε) deutlich höher als in bekannten Verbindungen mit gleichen Endgruppen und vergleichbar niedriger optischer Anisotropie. Die erfindungsgemässen Verbindungen besitzen zudem eine gute chemische und photochemische Stabilität und sind in Flüssigkristallmischungen löslich, ohne die Viskosität und die Mesophase dieser Mischungen zu beeinträchtigen. Die bei diesen Verbindungen auftretende S_{B}-Mesophase lässt sich in nematischen Mischungen leicht unterdrücken. Die erfindungsgemässen Verbindungen weisen im Gegensatz zu vorbekannten Verbindungen mit einer hohen dielektrischen Anisotropie eine sehr geringe elektrische Leitfähigkeit auf. Diese Eigenschaften bewirken, dass die Verwendung solcher Verbindungen zu Mischungen mit sehr niedriger Schwellenspannung (V₁₀), niedriger optischer Anisotropie und breiten nematischen Phasen führen. Sie eignen sich deshalb ganz besonders für die Verwendung in TN-Zellen (im 1. Minimum betrieben), OMI- und STN-Zellen und vor allem auch für die Verwendung in aktiv adressierten Anzeigevorrichtungen.

Der Ausdruck "cyclische oder acyclische hydrophobe Reste" für A und B bedeutet im Rahmen der vorliegenden Erfindung Reste wie
-R¹, 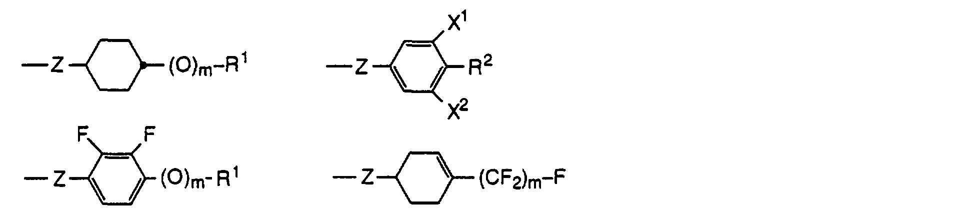 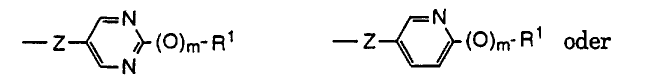 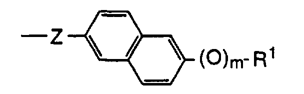 worin
- Z: eine Einfachbindung, -CH₂CH₂- oder -(CH₂)₄-;
- X¹ und X²: unabhängig voneinander Wasserstoff oder Fluor;
- m: 0 oder 1;
- R¹: Alkyl, Alkenyl oder Alkoxyalkyl mit höchstens 12 Kohlenstoffatomen, welche gegebenenfalls einfach oder mehrfach mit Fluor und/oder Chlor substituiert sein können: und
- R²: R¹, -O-R¹, Fluor, Chlor, Cyano oder Isothiocyanato bedeuten.

"Alkyl" bedeutet geradkettige oder verzweigte, gegebenenfalls chirale Reste mit 1 bis 12 Kohlenstoffatomen. Beispiele solcher Reste sind Methyl, Ethyl, Propyl, Butyl, Pentyl, Hexyl, Heptyl, Isopropyl, 2-Methyl-butyl, 2-Methyl-pentyl, 2-Methyl-hexyl, 3-Methyl-butyl, 3-Methyl-pentyl, 3-Methylhexyl, 4-Methyl-pentyl, 4-Methyl-hexyl und 5-Methyl-hexyl.

"Alkoxyalkyl" bedeutet geradkettige oder verzweigte, gegebenenfalls chirale Reste mit 2 bis 12 Kohlenstoffatomen. Beispiele solcher Reste sind Methoxymethyl, 2-Methoxyethyl, 3-Methoxypropyl, 4-Methoxybutyl, 5-Methoxypentyl, Ethoxymethyl, 2-Ethoxyethyl, 3-Ethoxypropyl, 4-Ethoxybutyl, Propyloxymethyl, 2-Propyloxyethyl, 3-Propyloxypropyl, Butyloxymethyl und 2-Butyloxyethyl.

"Alkenyl" bedeutet geradkettige oder verzweigte, gegebenenfalls chirale Reste mit 2 bis 12 Kohlenstoffatomen, worin die Doppelbindung entweder in 1-, 2-, 3- oder 4-Stellung oder endständig ist. Beispiele solcher Reste sind 1E-Alkenyl, wie 1E-Propenyl, 1E-Butenyl, 1E-Pentenyl, 1E-Hexenyl und 1E-Heptenyl; 2E-Alkenyl, wie 2E-Butenyl, 2E-Pentenyl, 2E-Hexenyl und 2E-Heptenyl; 3E-Alkenyl, wie 3E-Pentenyl, 3E-Hexenyl und 3E-Heptenyl; 4-Alkenyl, wie 4-Hexenyl, 4-Heptenyl; oder Alkenyl mit endständiger Doppelbindung, wie Vinyl, Allyl, 3-Butenyl, 4-Pentenyl, 5-Hexenyl und 6-Heptenyl.

Im Ausdruck "Alkyl, Alkenyl oder Alkoxyalkyl mit höchstens 12 Kohlenstoffatomen, welche gegebenenfalls einfach oder mehrfach mit Fluor und/oder Chlor substituiert sind" können diese Reste einfach oder mehrfach mit Fluor und/oder Chlor substituiert sein. Solche substituierte Reste sind beispielsweise Fluormethyl, Difluormethyl, Trifluormethyl, Chlordifluormethyl, 2-Fluorethyl, 2,2-Difluorethyl, 2,2,2-Trifluorethyl, Perfluorethyl, Perfluorpropyl, E-Chlorvinyl, E-Fluorvinyl, 2,2-Difluorvinyl und dergleichen.

Besonders bevorzugt sind geradkettige Alkyl-, Alkenyl- oder Alkoxyalkyl-Reste mit 1 bzw. 2 bis 7 Kohlenstoffatomen.

Besonders bevorzugte Reste R¹ sind Propyl, Butyl, Pentyl, Difluormethyl, Trifluormethyl, Vinyl, E-Propenyl, lE-Butenyl, 1E-Pentenyl, 3-Butenyl, 3E-Pentenyl, E-Fluorvinyl, 2,2-Difluorvinyl oder E-Chlorvinyl.

Bevorzugte Verbindungen der allgemeinen Formel I sind diejenigen der Formeln 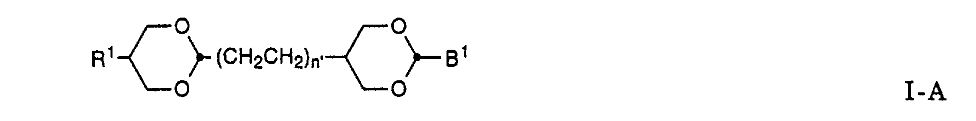 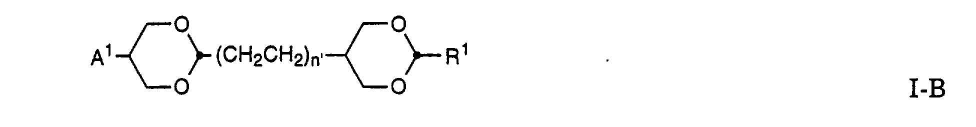 worin
- R¹: die obengenannten Bedeutungen hat;
- A¹: Alkyl, Alkenyl, Alkoxyalkyl oder eine Gruppe der Formel 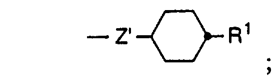 und
- B¹: Alkyl, Alkenyl, Alkoxyalkyl oder eine Gruppe der Formel 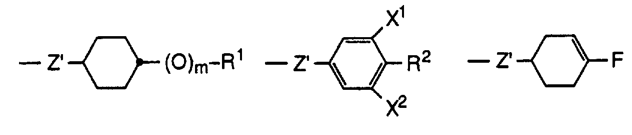 oder 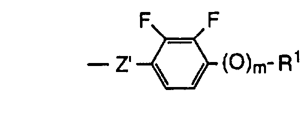 bedeuten;
worin
- n': 0 oder 1;
- m: 0 oder 1;
- Z': eine Einfachbindung oder -CH₂CH₂- bedeuten; und
- R²: die oben angegebenen Bedeutungen hat.

Besonders bevorzugte Verbindungen der Formel I-A sind somit Verbindungen der Formeln 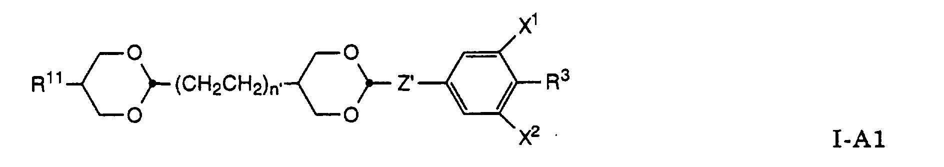 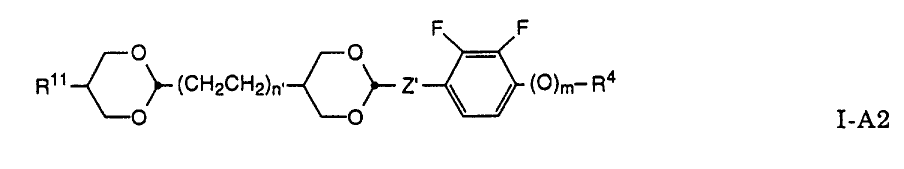 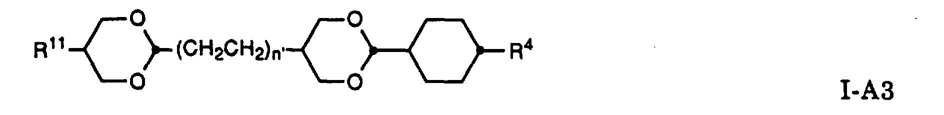 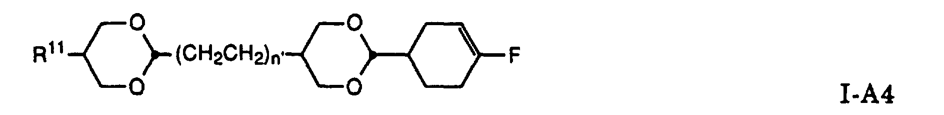 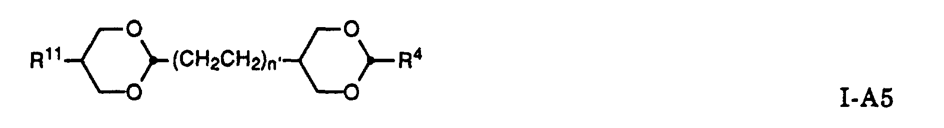 worin
- R¹¹: Alkyl oder Alkenyl mit höchstens 7 Kohlenstoffatomen;
- n': 0 oder 1;
- m: 0 oder 1;
- X¹ und X²: unabhängig voneinander Wasserstoff oder Fluor;
- Z': eine Einfachbindung oder -CH₂CH₂-;
- R³: Alkoxy, Difluormethoxy, Trifluormethoxy, Chlordifluormethoxy, 2,2,2-Trifluorethyloxy, Fluor, Chlor oder Cyano; und
- R⁴: Alkyl, Alkoxyalkyl oder Alkenyl mit höchstens 7 Kohlenstoffatomen bedeuten.

Bevorzugte Verbindungen der Folmel I-A5, worin n' = 1 ist, sind beispielsweise.
trans-2-Ethyl-5-[2-(trans-5-propyl-1,3-dioxan-2-yl)-ethyl]-1,3-dioxan,
trans-2-Ethyl-5-[2-(trans-5-pentyl-1,3-dioxan-2-yl)-ethyl]-1,3-dioxan,
trans-2-Propyl-5-[2-(trans-5-propyl-1,3-dioxan-2-yl)-ethyl]-1,3-dioxan
trans-2-Propyl-5-[2-(trans-5-butyl-1,3-dioxan-2-yl)-ethyl]-1,3-dioxan,
trans-2-Propyl-5-[2-(trans-5-pentyl-1,3-dioxan-2-yl)-ethyl]-1,3-dioxan
(E)-trans-2-Ethyl-5-[2-(trans-5-propenyl-1,3-dioxan-2-yl)-ethyl]-1,3-dioxan,
(E)-trans-2-Propyl-5-[2-(trans-5-propenyl-1,3-dioxan-2-yl)-ethyl]-1,3-dioxan und
(E)-trans-2-Propyl-5-[2-(trans-5-but-1-enyl-1,3-dioxan-2-yl)-ethyl]-1,3-dioxan.

Bevorzugte Verbindungen der Formel I-A5, worin n' = 0 ist, sind beispielsweise
trans-2-Butyl-5-(trans-5-propyl-1,3-dioxan-2-yl)-1,3-dioxan,
trans-2-Butyl-5-(trans-5-butyl-1,3-dioxan-2-yl)-1,3-dioxan,
trans-2-Butyl-5-(trans-5-pentyl-1,3-dioxan-2-yl)-1,3-dioxan,
(E)-trans-2-Butyl-5-(trans-5-propenyl-1,3-dioxan-2-yl)-1,3-dioxan,
(E)-trans-2-Butyl-5-(trans-5-but-1-enyl-1,3-dioxan-2-yl)-1,3-dioxan,
trans-5-Butyl-trans-2'-propyl-2,5'-bi-1,3-dioxan,
trans,trans-5,2'-Dipropyl-2,5'-bi-1,3-dioxan und
trans,trans-5,2'-Dipentyl-2,5'-bi-1,3-dioxan.

Bevorzugte Verbindungen der Formel I-A3, worin n' = 1 ist. sind beispielsweise
trans-2-(trans-4-Butylcyclohexyl)-5-[2-(trans-5-propyl-1,3-dioxan-2-yl)-ethyl]-1,3-dioxan, trans-2-(trans-4-Butylcyclohexyl)-5-[2-(trans-5-butyl-1,3-dioxan-2-yl)-ethyl]-1,3-dioxan, trans-2-(trans-4-Butylcyclohexyl)-5-[2-(trans-5-pentyl-1,3-dioxan-2-yl)-ethyl]-1,3-dioxan (E)-trans-2-(trans-4-Butylcyclohexyl)-5-[2-(trans-5-propenyl-1,3-dioxan-2-yl)-ethyl]-1,3-dioxan und (E)-trans-2-(trans-4-Butylcyclohexyl)-5-[2-(trans-5-but-1-enyl-1,3-dioxan-2-yl)-ethyl]-1,3-dioxan.

Bevorzugte Verbindungen der Formel I-A1, worin n' = 1 ist und Z' eine Einfachbindung bedeutet, sind beispielsweise
trans-2-(3,4-Difluorphenyl)-5-[2-(trans-5-propyl-1,3-dioxan-2-yl)-ethyl]-1,3-dioxan
trans-2-(3,4-Difluorphenyl)-5-[2-(trans-5-butyl-1,3-dioxan-2-yl)-ethyl]-1,3-dioxan,
(E)-trans-2-(3,4-Difluorphenyl)-5-[2-(trans-5-propenyl-1,3-dioxan-2-yl)-ethyl]-1,3-1,3-dioxan
trans-2-(3,4,5-Trifluorphenyl)-5-[2-(trans-5-propyl-1,3-dioxan-2-yl)-ethyl]-1,3-dioxan
(E)-trans-2-(3,4,5-Trifluorphenyl)-5-[2-(trans-5-propenyl-1,3-dioxan-2-yl)-ethyl]-1,3-dioxan
trans-2-(4-Trifluormethyl-phenyl)-5-[2-(trans-5-propyl-1,3-dioxan-2-yl)-ethyl]-1,3-dioxan,
trans-2-(4-Trifluormethyl-phenyl)-5-[2-(trans-5-butyl-1,3-dioxan-2-yl)-ethyl]-1,3-dioxan und
(E)-trans-2-(4-Trifluormethyl-phenyl)-5-[2-(trans-5-propenyl-1,3-dioxan-2-yl)-ethyl]-1,3-dioxan.

Bevorzugte Verbindungen der Formel I-A1, worin n' = 1 ist und Z' -CH₂CH₂- bedeutet, sind beispielsweise
trans-2-(4-Trifluormethoxy-phenethyl)-5-[2-(trans-5-butyl-1,3-dioxan-2-yl)-ethyl]-1,3-dioxan und
(E)-trans-2-(3,4-Difluor-phenethyl)-5-[2-(trans-5-but-1-enyl-1,3-dioxan-2-yl)-ethyl]-1,3-dioxan.

Bevorzugte Verbindungen der Formel I-A1, worin n' = 0 ist und Z' eine Einfachbindung bedeutet, sind beispielsweise
trans-5-Propyl-trans-2'-(4-trifluormethyl-phenyl)-2,5'-bi-1,3-dioxan,
trans-5-Butyl-trans-2'-(4-trifluormethyl-phenyl)-2,5'-bi-1,3-dioxan,
trans-5-Pentyl-trans-2'-(4-trifluormethyl-phenyl)-2,5'-bi-1,3-dioxan,
trans-5-Propyl-trans-2'-(4-trifluormethoxy-phenyl)-2,5'-bi-1,3-dioxan,
trans-5-Butyl-trans-2'-(4-trifluormethoxy-phenyl)-2,5'-bi-1,3-dioxan,
trans-5-Pentyl-trans-2'-(4-trifluormethoxy-phenyl)-2,5'-bi-1,3-dioxan,
trans-5-Propyl-trans-2'-(3,4-difluor-phenyl)-2,5'-bi-1,3-dioxan,
trans-5-Butyl-trans-2'-(3,4-difluor-phenyl)-2,5'-bi-1,3-dioxan,
trans-5-Pentyl-trans-2'-(3,4-difluor-phenyl)-2,5'-bi-1,3-dioxan,
   trans-5-Propyl-trans-2'-(3,4,5-trifluor-phenyl)-2,5'-bi-1,3-dioxan,
trans-5-Butyl-trans-2'-(3,4,5-trifluor-phenyl)-2,5'-bi-1,3-dioxan und
trans-5-Pentyl-trans-2'-(3,4,5-trifluor-phenyl)-2,5'-bi-1,3-dioxan.

Bevorzugte Verbindungen der Formel I-A1, worin n' = 0 ist und Z' -CH₂CH₂- bedeutet sind beispielsweise
trans-2'-(3,4-Difluor-phenethyl)-trans-5-butyl-2,5'-bi-1,3-dioxan,
trans-5-Butyl-trans-2'-(4-trifluormethyl-phenethyl)-2,5'-bi-1,3-dioxan,
trans-5-Butyl-trans-2'-(3,4-difluor-phenethyl)-2,5'-bi-1,3-dioxan und
trans-5-Pentyl-trans-2'-(3,4-difluor-phenethyl)-2,5'-bi-1,3-dioxan.

Besonders bevorzugte Verbindungen der Formel I-B sind 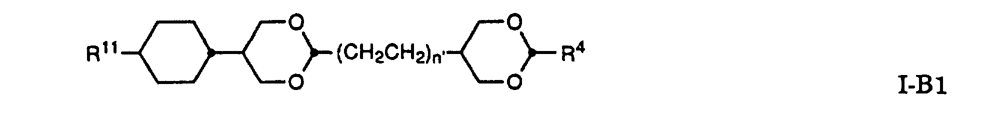 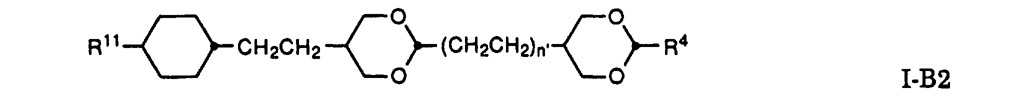 worin die Symbole die oben angegebenen Bedeutungen haben.

Die erfindungsgemässen Verbindungen der Formel I können in an sich bekannter Weise hergestellt werden. Die Synthese wird in Schema 1 veranschaulicht. So können 2,5'-Bi-1,3-dioxane der Formel I, worin n = 0 ist, ausgehend von einem Alkoxymethyliden-malonester (**2b**) und einem entsprechend substituierten 1,3-Propandiol (**1**) über einen Dioxanylmalonester (**5**, n = 0) hergestellt werden. Die Umsetzung des Alkoxymethylidenmalonesters (**2b**) mit dem 1,3-Propandiol (**1**) kann z. B. in einem inerten Lösungsmittel in Gegenwart einer katalytischen Menge einer Säure, wie p-Toluolsulfonsäure (TsOH) erfolgen.
Di-1,3-dioxane-Derivate der Formel I, worin n = 1 oder 2 ist, können über das Zwischenprodukt **3**, worin n = 1 bzw. 2 ist, hergestellt werden, beispielsweise durch Umacetalisierung eines entsprechend substituierten Bromalkyldioxolans (**2a**) zum Dioxan (**3**) (als cis/trans Gemisch), welches durch anschliessende Alkylierung eines Alkalisalzes von Dialkylmalonat (**4**) in den Malonester (**5**, n = 1 oder 2) übergeführt werden kann.
Die Zwischenprodukte **5** (sowohl n = 0 als auch n = 1 oder 2) können in einem inerten Lösungsmittel beispielsweise mit Lithiumaluminiumhydrid zu den entsprechenden Diolen (**6**) reduziert werden, wobei die Aufarbeitung vorzugsweise im basischen Milieu erfolgt. In der Schlusstufe können diese Diole (**6**) mit entsprechend substituierten Aldehyden (**7**) in einem inerten Lösungsmittel bei ca. 50 - 110°C und in Gegenwart eines sauren Katalysators (z.B. p-Toluolsulfonsäure, verdünnte Schwefelsäure oder Pyridinium-p-toluolsulfonat) umgesetzt werden. Für Verbindungen der Formel I, worin Z -CH₂CH₂- bedeutet, können die Diole (**6**) mit entsprechenden Derivaten der Aldehyde (**7**), beispielsweise einem Enolether oder einem Acetal, umgesetzt werden. Die Reaktionszeit und die Säurekonzentration sind, wegen der möglichen Oeffnung des Dioxanringes des Diols (6) einerseits und wegen der für hohe Ausbeuten an all-trans-Verbindungen erforderlichen Erreichung des Gleichgewichts andererseits, kritisch. Das all-trans Isomere der Formel I kann durch Chromatographie des Isomerengemisches und anschliessende Umkristallisation isoliert werden.

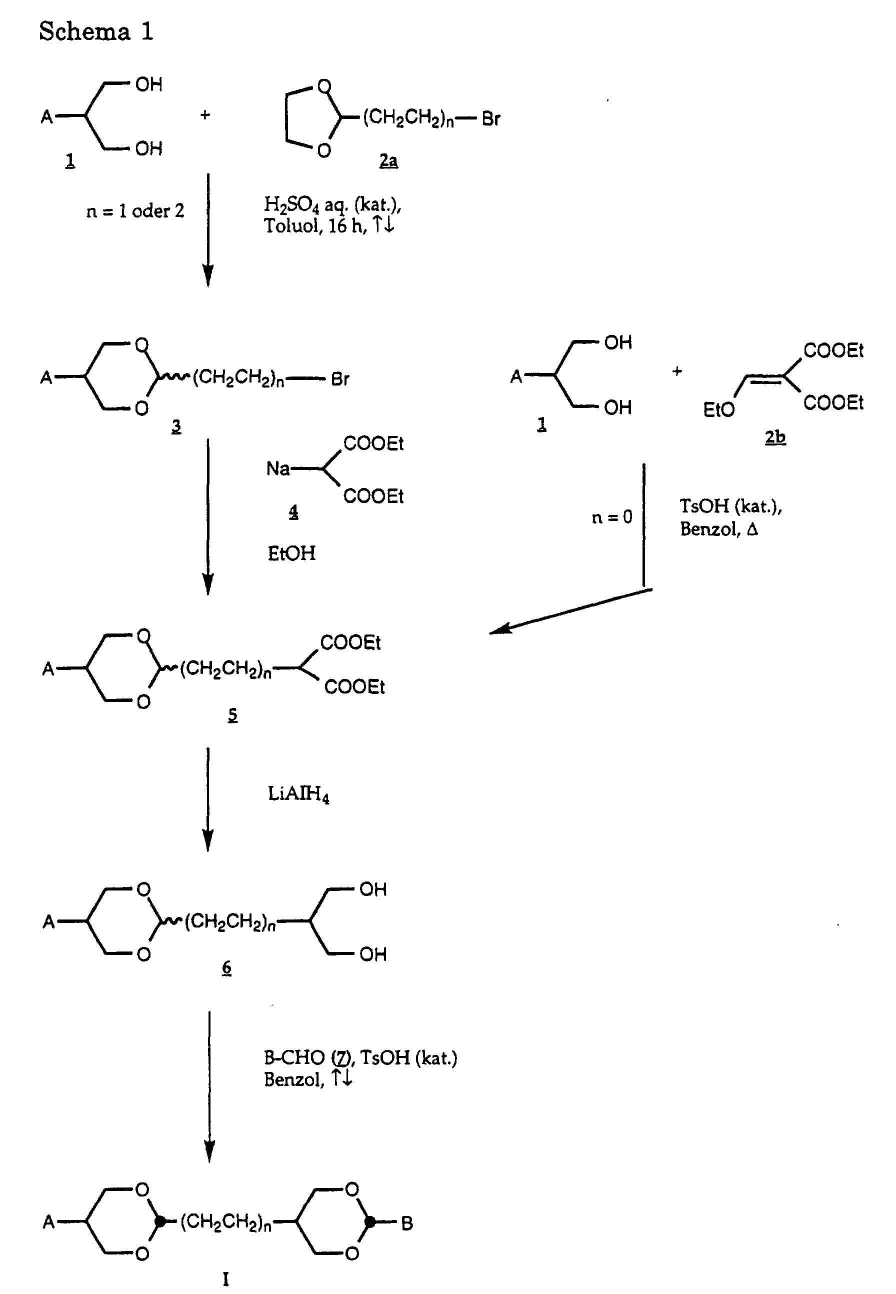

Die im Schema verwendeten Symbole haben die oben angegebenen Bedeutungen.

Die Verbindungen der Formel I können in Form von Gemischen untereinander und/oder mit anderen Flüssigkristallkomponenten verwendet werden. Geeignete Flüssigkristallkomponenten sind dem Fachmann in grosser Zahl bekannt, z.B. aus D. Demus et al., Flüssige Kristalle in Tabellen, VEB Deutscher Verlag für Grundstoffindustrie, Leipzig, Bände I und II, und viele davon sind zudem im Handel erhältlich.

Die Erfindung betrifft ebenfalls flüssigkristalline Gemische mit mindestens 2 Komponenten, welche dadurch gekennzeichnet sind, dass mindestens eine Komponente eine Verbindung der Formel I ist. Eine zweite und gegebenenfalls weitere Komponenten können weitere Verbindungen der allgemeinen Formel I oder andere geeignete Flüssigkristallkomponenten sein.

Aufgrund der guten Löslichkeit der erfindungsgemässen Verbindungen der Formel I in anderen Flüssigkristallmaterialien und aufgrund ihrer guten Mischbarkeit untereinander kann der Anteil an Verbindungen der Formel I in den erfindungsgemässen Gemischen relativ hoch sein und beispielsweise 1-30 Gew.-% betragen. Im allgemeinen ist ein Anteil von etwa 1-25 Gew.-%, insbesondere von etwa 3-20 Gew.-%, an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der Formeln: 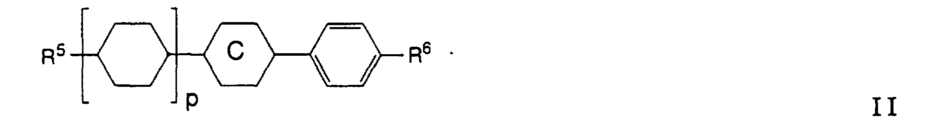 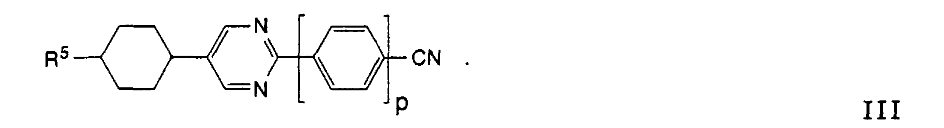 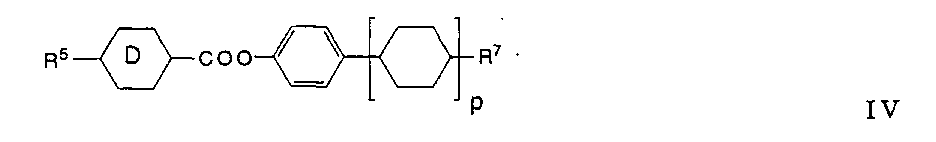 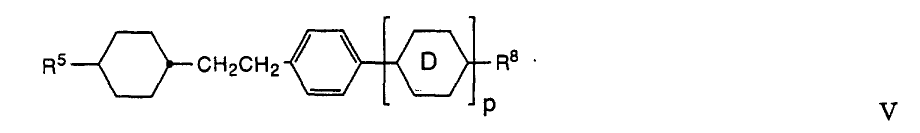 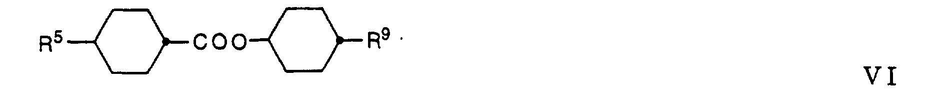 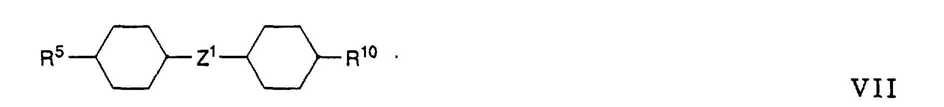 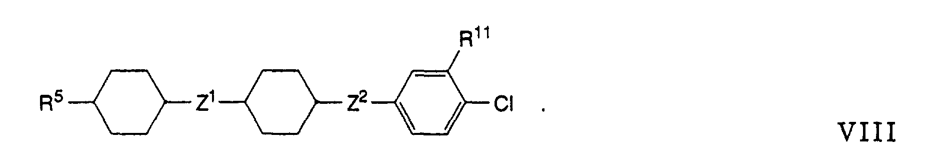 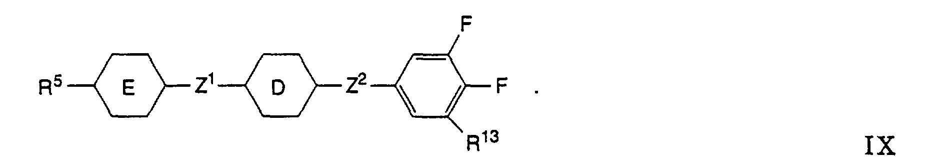 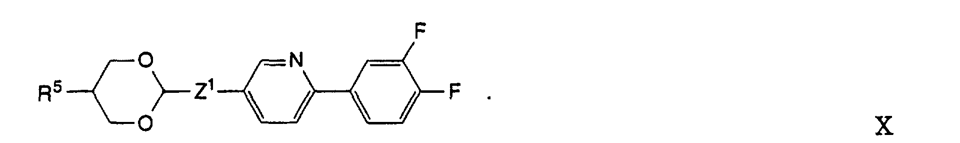 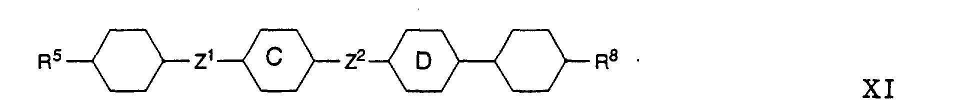 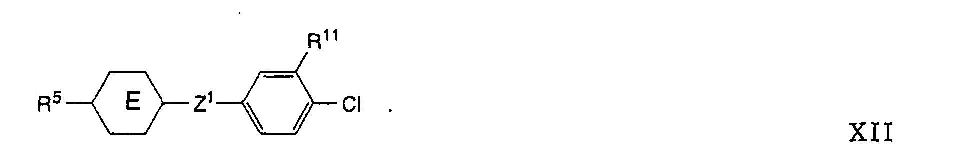 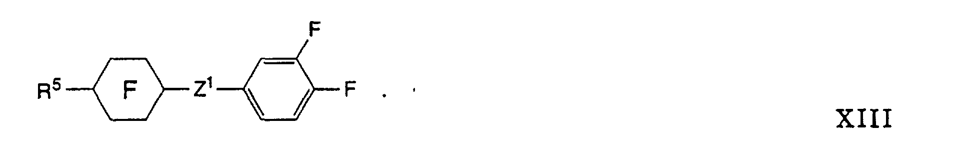 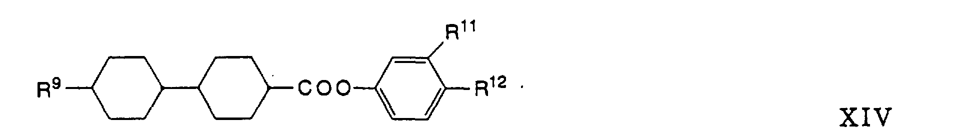 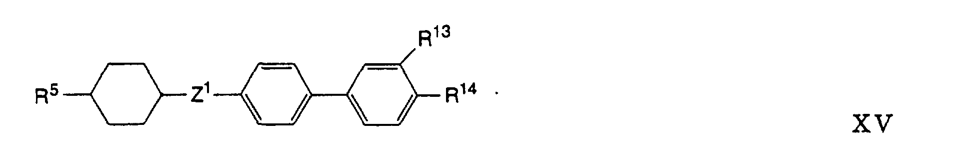 und 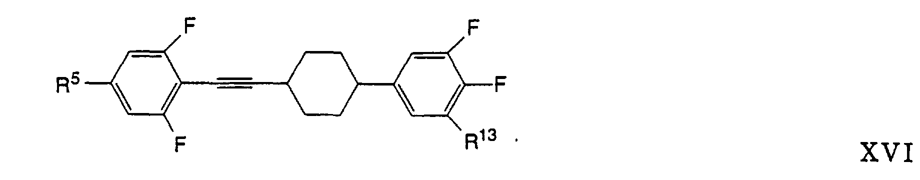 worin
- R⁵, R⁷: unabhängig voneinander Alkyl, Alkoxyalkyl, 3E-Alkenyl, 4-Alkenyl oder an gesättigten Ringen auch 1E-Alkenyl;
- p: 0 oder 1;
- Ring C: 1,4-Phenylen, Pyridin-2,5-diyl, Pyrimidin-2,5-diyl, trans-1,4-Cyclohexylen oder trans-1,3-Dioxan-2,5-diyl;
- R⁶: Cyano, Isothiocyanato, Fluor, Alkyl, 3E-Alkenyl, 4-Alkenyl, Alkoxy, 2E-Alkenyloxy, 3-Alkenyloxy oder 1-Alkinyl;
- Ring D: 1,4-Phenylen oder trans-1,4-Cyclohexylen;
- R⁸: Alkyl, 3E-Alkenyl, 4-Alkenyl, oder an trans-1,4-Cyclohexylen auch 1E-Alkenyl, oder an 1,4-Phenylen auch Cyano, Isothiocyanato, Alkoxy, 2E-Alkenyloxy oder 3-Alkenyloxy;
- R⁹: Alkyl, 1E-Alkenyl, 3E-Alkenyl oder 4-Alkenyl;
- R¹⁰: Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl;
- Z¹, Z²: unabhängig voneinander eine einfache Kovalenzbindung oder -CH₂CH₂-, wobei zwei aromatische Ringe immer durch eine einfache Kovalenzbindung verknüpft sind;
- Ring E: trans-1,4-Cyclohexylen oder trans-1,3-Dioxan-2,5-diyl;
- R¹¹: Wasserstoff, Fluor oder Chlor;
- R¹²: Cyano, Fluor oder Chlor;
- R¹³: Wasserstoff oder Fluor;
- R¹⁴: Fluor oder Chlor, und.
- Ring F: Pyrimidin-2,5-diyl, trans-1,4-Cyclohexylen oder trans-1,3-Dioxan-2,5-diyl bedeuten.

Die im Zusammenhang mit den Verbindungen der Formeln II bis XVI verwendeten Ausdrücke sind wie weiter vorne definiert.
Der Ausdruck "aromatische Ringe" bezeichnet Ringe, wie beispielsweise 1,4-Phenylen, Pyridin-2,5-diyl oder Pyrimidin-2,5-diyl.
Der Ausdruck "gesättigte Ringe" bezeichnet trans-1,4-Cyclohexylen oder trans-1,3-Dioxan-2,5-diyl.
Der Ausdruck "Alkoxy" bedeutet geradkettige oder verzweigte, gegebenenfalls chirale Reste mit 1 bis 12 Kohlenstoffatomen, vorzugsweise geradkettige Reste, wie beispielsweise Methoxy, Ethoxy, Propyloxy, Butyloxy, Pentyloxy, Hexyloxy, Heptyloxy und dergleichen.
"2E- bzw. 3-Alkenyloxy" bedeutet in diesem Zusammenhang vorzugsweise geradkettige Alkenyloxyreste mit 3. bzw. 4 bis 12 Kohlenstoffatomen, in denen sich die Doppelbindung in 2- bzw. 3-Stellung befindet und E bzw. Z die bevorzugte Konfiguration angibt, wie beispielsweise Allyloxy, 2E-Butenyloxy, 2E-Pentenyloxy, 2E-Hexenyloxy, 2E-Heptenyloxy, 3-Butenyloxy, 3-Pentenyloxy, 3-Hexenyloxy, 3-Heptenyloxy, 4-Pentenyloxy, 5-Hexenyloxy, 6-Heptenyloxy und dergleichen.
"1-Alkinyl" bedeutet in diesem Zusammenhang vorzugsweise geradkettige Alkinylreste mit 2 bis 12 Kohlenstoffatomen, , in denen sich die Dreifachbindung in 1-Stellung befindet, wie beispielsweise Ethinyl, 1-Propinyl, 1-Butinyl, 1-Pentinyl und dergleichen.

Die erfindungsgemässen Mischungen können ferner optisch aktive Verbindungen (z.B. optisch aktive 4'-Alkyl- oder 4'-Alkoxy-4-biphenylcarbonitrile) und/oder dichroitische Farbstoffe (z.B. Azo-, Azoxy- oder Anthrachinonfarbstoffe) enthalten. Der Anteil solcher Verbindungen wird durch die Löslichkeit, die gewünschte Ganghöhe der Helix, Farbe, Extinktion und dergleichen bestimmt. Im allgemeinen beträgt der Anteil optisch aktiver Verbindungen und dichroitischer Farbstoffe höchstens je etwa 10 Gew.% im Gesamtgemisch.

Die Herstellung der flüssigkristallinen Gemische und der elektro-optischen Vorrichtung kann in an sich bekannter Weise erfolgen.

Die Herstellung der Verbindungen der Formel I sowie flüssigkristalline Gemische enthaltend diese Verbindungen werden durch die folgenden Beispiele weiter veranschaulicht. C bedeutet eine kristalline, S eine smektische, N die nematische und I die isotrope Phase. V₁₀ bezeichnet die Spannung für 10% Transmission (Blickrichtung senkrecht zur Plattenoberfläche). tₒₙ und t_{off} bezeichnen die Einschaltzeit bzw. die Ausschaltzeit, Δn die optische Anisotropie, und GC bedeutet Gaschromatographie.

### Beispiel 1

Eine Lösung aus 1,50 g (E)-2-[2-(5-But-1-enyl-1,3-dioxan-2-yl)-ethyl]-1,3-propandiol und 2 ml Butyraldehyd in 40 ml Benzol wurde mit 28,5 mg p-Toluolsulfonsäure-monohydrat versetzt und 40 Minuten bei 68°C gerührt. Das Reaktionsgemisch wurde mit einigen Tropfen Triethylamin neutralisiert und nach dem Erkalten mit gesättigter Kochsalzlösung gewaschen, über Natriumsulfat getrocknet und eingeengt. Das Rohprodukt (2,15 g) wurde an 40 g Kieselgel mit Hexan/Ethylacetat 5% (v/v) chromatographiert. Es resultierten 1,40 g Isomerengemisch mit einem trans/trans-Anteil von 52%. Zweimalige Umkristallisation aus Hexan ergab 0,41 g reines (E)-trans-2-Propyl-5-[2-(trans-5-but-1-enyl-1,3-dioxan-2-yl)-ethyl]-1,3-dioxan, Smp. (C-S_{B}) 42°C, Klp. (S_{B}-I) 117,8°C.

Das aus Ausgangsmaterial verwendete (E)-2-[2-(5-But-1-enyl-1,3-dioxan-2-yl)-ethyl]-1,3-propandiol wurde wie folgt hergestellt:
a) Eine Lösung aus 14,4 ml 2-(2-Bromethyl)-dioxolan und 20,6 g (E)-2-But-1-enyl-1,3-propandiol in 200 ml Toluol wurde mit 2,7 ml 10%iger wässriger Schwefelsäure (v/v) und 8 ml Wasser über Nacht zu leichtem Sieden erhitzt. Das Gemisch wurde mit 6 ml Triethylamin versetzt, die wässrige Phase abgetrennt. Die organische Phase wurde mit halbkonz. Kochsalzlösung gewaschen, über Natriumsulfat getrocknet, filtriert und eingeengt. Das erhaltene orange Oel (31,3 g) wurde an 400 g Kieselgel mit Hexan/Ethylacetat 2,5% (v/v) chromatographiert. Es resultierten 23,4 g (E)-2-(2-Bromethyl)-5-but-1-enyl-1,3-dioxan (GC-Reinheit 97,5% trans/cis-Verhältnis 73:27) als gelbliches Oel.
b) Zu einer Lösung von 13,0 ml Diethylmalonat in 40 ml Ethanol wurde eine aus 2,3 g Natrium in 60 ml Ethanol frisch bereitete Natriumethylat-Lösung getropft, nach 45 Minuten Rühren wurden 23,4 g (E)-2-(2-Brom-ethyl)-5-but-1-enyl-1,3-dioxan zugetropft. Das Reaktionsgemisch wurde über Nacht stehen gelassen und danach 30 Minuten zum Sieden erhitzt. Nach dem Erkalten wurde zunächst mit 150 ml Diethylether verdünnt und mit 100 ml halbkonz. und dann mit 50 ml konz. Kochsalz-Lösung gewaschen, über Natriumsulfat getrocknet, filtriert und eingeengt. Das erhaltene orangerote Oel (29,5 g) wurde zur Trennung an 300 g Kieselgel mit Hexan/Ethylacetat 5% (v/v) chromatographiert. Die produkthaltigen Fraktionen (24,0 g) wurden zur Abtrennung der dialkylierten Nebenprodukte destilliert. Die Fraktion mit Sdp. 130-150°/ca. 0,01 Torr wog 16,75 g und war nach GC zu 95% ein trans/cis-Gemisch von (E)-[2-(5-But-1-enyl-1,3-dioxan-2-yl)-ethyl]-malon-säurediethylester.
c) Unter Schutzgas wurde eine Lösung von 16,75 g (E)-[2-(5-But-1-enyl-1,3-dioxan-2-yl)-ethyl]-malonsäurediethylester in 25 ml Tetrahydrofuran bei 5-8°C zu einer Suspension von 3,87 g Lithiumaluminiumhydrid in 100 ml Tetrahydrofuran getropft. Das Reaktionsgemisch wurde noch 15 Minuten bei 5°C, dann 2 Stunden bei Raumtemperatur und schliesslich 1,2 Stunden bei Siedetemperatur gerührt. Nach dem Erkalten wurde vorsichtig 3N Natronlauge zugetropft, bis sich ein fester weisser Klumpen bildete. Die Lösung wurde dekantiert und der Rückstand zweimal mit Diethylether gewaschen. Die organischen Phasen wurden vereinigt, nacheinander mit 25 ml konz. Natriumbicarbonat-Lösung und zweimal je 25 ml konz. Kochsalz-Lösung gewaschen, über Natriumsulfat getrocknet, filtriert und das Filtrat eingeengt. Es resultierten 12,20 g rohes (E)-2-[2-(5-But-1-enyl-1,3-dioxan-2-yl)-ethyl]-1,3-propandiol als klebrig-festes Rohprodukt, Smp. bis 87,1°C (GC-Reinheit 86%, trans/cis-Verhältnis 7:3), das ohne weitere Reinigung in der Schlusstufe eingesetzt wurde.

In analoger Weise können hergestellt werden:
trans-2-Ethyl-5-[2-(trans-5-propyl-1,3-dioxan-2-yl)-ethyl]-1,3-dioxan, Smp. (C-S_{B}) 56,8°C, Klp. (S_{B}-I) 75,1°C;
trans-2-Ethyl-5-[2-(trans-5-butyl-1,3-dioxan-2-yl)-ethyl]-1,3-dioxan;
trans-2-Ethyl-5-[2-(trans-5-pentyl-1,3-dioxan-2-yl)-ethyl]-1,3-dioxan, Smp. (C-S_{B}) 40,0°C, Klp. (S_{B}-I) 91°C;
(E)-trans-2-Ethyl-5-[2-(trans-5-propenyl-1,3-dioxan-2-yl)-ethyl]-1,3-dioxan, Smp. (C-S_{B}) 68°C, Klp. (S_{B}-I) 76,6°C;
trans-2-Propyl-5-[2-(trans-5-propyl-1,3-dioxan-2-yl)-ethyl]-1,3-dioxan, Smp. (C-S_{B}) 58°C, Klp. (S_{B}-I) 98,3°C;
trans-2-Propyl-5-[2-(trans-5-butyl-1,3-dioxan-2-yl)-ethyl]-1,3-dioxan, Smp. (C-S_{B}) 62,5°C, Klp. (S_{B}-I) 110°C;
trans-2-Propyl-5-[2-(trans-5-pentyl-1,3-dioxan-2-yl)-ethyl]-1,3-dioxan, Smp. (C-S_{B}) 57,5°C, Klp. (S_{B}-I) 113,1°C;
trans-2-Propyl-5-[2-(trans-5-hexyl-1,3-dioxan-2-yl)-ethyl]-1,3-dioxan;
(E)-trans-2-Propyl-5-[2-(trans-5-propenyl-1,3-dioxan-2-yl)-ethyl]-1,3-dioxan, Smp. (C-S_{B}) 68,7°C, Klp. (S_{B}-I) 100,5°C;
(E)-trans-2-Propyl-5-[2-(trans-5-pent-1-enyl-1,3-dioxan-2-yl)-ethyl]-1,3-dioxan;
trans-2-Propyl-5-[trans-5-but-3-enyl-1,3-dioxan-2-yl)-ethyl]-1,3-dioxan;
trans-2-Propyl-5-[trans-5-(3-methoxy-propyl)-1,3-dioxan-2-yl]-1,3-dioxan;
trans-2-Butyl-5-[trans-5-propyl-1,3-dioxan-2-yl)-1,3-dioxan, Smp. (C-S_{B}) 61,5°C, Klp. (S_{B}-I) 102,8°C;
trans-2-Butyl-5-(trans-5-butyl-1,3-dioxan-2-yl)-1,3-dioxan, Smp. (C-S_{B}) 78,5°C, Klp. (S_{B}-I) 112°C;
trans-2-Butyl-5--(trans-5-pentyl-1,3-dioxan-2-yl)-1,3-dioxan, Smp. (C-S_{B}) 65°C, Klp. (S_{B}-I) 116,3°C;
(E)-trans-2-Butyl-5-(trans-5-propenyl-1,3-dioxan-2-yl)-1,3-dioxan, Smp. (C-S_{B}) 45,5°C, Klp. (S_{B}-I) 106,8°C;
(E)-trans-2-Butyl-5-(trans-5-but-1-enyl-1,3-dioxan-2-yl)-1,3-dioxan, Smp. (C-S_{B}) 42°C, Klp. (S_{B}-I) 120,5°C;
(E)-trans-2-Butyl-5-(trans-5-pent-1-enyl-1,3-dioxan-2-yl)-1,3-dioxan;
trans-2-Pentyl-5-(trans-5-propyl-1,3-dioxan-2-yl)-1,3-dioxan;
trans-2-Pentyl-5-(trans-5-butyl-1,3-dioxan-2-yl)-1,3-dioxan;
trans-2-Pentyl-5-[2-(trans-5-pentyl-1,3-dioxan-2-yl)-ethyl]-1,3-dioxan;
(E)-trans-2-Pentyl-5-[2-(trans-5-but-1-enyl-1,3-dioxan-2-yl)-ethyl]-1,3-dioxan;
(E)-trans-2-Pentyl-5-[2-(trans-5-pent-1-enyl-1,3-dioxan-2-yl)-ethyl]-1,3-dioxan;
trans-2-Pentyl-5- [2-(trans-5-but-3-enyl-1,3-dioxan-2-yl)-ethyl]-1,3-dioxan;
(E)-trans-2-Pentyl-5-[2-(trans-5-pent-3-enyl-1,3-dioxan-2-yl)-ethyl]-1,3-dioxan;
trans-2-Pentyl-5-[2-(trans-5-pent-4-enyl-1,3-dioxan-2-yl)-ethyl]-1,3-dioxan;
trans-2-Pentyl-5-[2-[trans-5-(3-methoxy-propyl)-1,3-dioxan-2-yl]-ethyl]-1,3-dioxan;
trans-2-(trans-4-Ethylcyclohexyl)-5-[2-(trans-5-propyl-1,3-dioxan-2-yl)-ethyl]-1,3-dioxan;
trans-2-(trans-4-Ethylcyclohexyl)-5-[2-(trans-5-pentyl-1,3-dioxan-2-yl)-ethyl]-1,3-dioxan;
trans-2-(trans-4-Propylcyclohexyl)-5-[2-(trans-5-propyl-1,3-dioxan-2-yl)-ethyl]-1,3-dioxan;
trans-2-(trans-4-Propylcyclohexyl)-5-[2-(trans-5-butyl-1,3-dioxan-2-yl)-ethyl]-1,3-dioxan;
trans-2-(trans-4-Propylcyclohexyl)-5-[2-(trans-5-pentyl-1,3-dioxan-2-yl)-ethyl]-1,3-dioxan;
(E)-trans-2-(trans-4-Propylcyclohexyl)-5-[2-(trans-5-propenyl-1,3-dioxan-2-yl)-ethyl]-1,3-dioxan;
(E)-trans-2-(trans-4-Propylcyclohexyl)-5-[2-(trans-5-but-1-enyl-1,3-dioxan-2-yl)-ethyl]-1,3-dioxan;
(E)-trans-2-(trans-4-Propylcyclohexyl)-5-[2-(trans-5-pent-1-enyl-1,3-dioxan-2-yl)-ethyl]-1,3-dioxan;
trans-2-(trans-4-Butylcyclohexyl)-5-[2-(trans-5-propyl-1,3-dioxan-2-yl)-ethyl]-1,3-dioxan, Smp. (C-S_{B}) 107°C, Klp. (S_{B}-I) 168,5°C;
trans-2-(trans-4-Butylcyclohexyl)-5-[2-trans-5-butyl-1,3-dioxan-2-yl)-ethyl]-1,3-dioxan, Smp. (C-S_{B}) 100°C, Klp. (S_{B}-I) 177°C;
trans-2-(trans-4-Butylcyclohexyl)-5-[2-(trans-5-pentyl-1,3-dioxan-2-yl)-ethyl]-1,3-dioxan, Smp. (C-S_{B}) 105°C, Klp. (S_{B}-I) 184°C;
(E)-trans-2-(trans-4-Butylcyclohexyl)-5-[2-(trans-5-propenyl-1,3-dioxan-2-yl)-ethyl]-1,3-dioxan, Smp. (C-S_{B}) 117,5°C, Klp. (S_{B}-I) 181,5°C;
(E)-trans-2-(trans-4-Butylcyclohexyl)-5-[2-(trans-5-but-1-enyl-1,3-dioxan-2-yl)-ethyl]-1,3-dioxan, Smp. (C-S_{B}) 105°C, Klp. (S_{B}-I) 191,5°C;
(E)-trans-2-(trans-4-Butylcyclohexyl)-5-[2-(trans-5-pent-1-enyl-1,3-dioxan-2-yl)-ethyl]-1,3-dioxan;
trans-2-(trans-4-Butylcyclohexyl)-5-[2-(trans-5-but-3-enyl-1,3-dioxan-2-yl)-ethyl] -1,3-dioxan;
trans-2-(trans-4-Pentylcyclohexyl)-5-[2-(trans-5-propyl-1,3-dioxan-2-yl)-ethyl]-1,3-dioxan;
trans-2-(trans-4-Pentylcyclohexyl)-5-[2-(trans-5-pentyl-1,3-dioxan-2-yl)-ethyl]-1,3-dioxan;
(E)-trans-2-(trans-4-Pentylcyclohexyl)-5-[2-(trans-5-propenyl-1,3-dioxan-2-yl)-ethyl]-1,3-dioxan;
(E)-trans-2-(trans-4-Pentylcyclohexyl)-5-[2-(trans-5-but-1-enyl-1,3-dioxan-2-yl)-ethyl]-1,3-dioxan;
trans-2-(4-Fluorphenyl)-5-[2-(trans-5-propyl-1,3-dioxan-2-yl)-ethyl]-1,3-dioxan;
trans-2-(4-Fluorphenyl)-5-[2-(trans-5-butyl-1,3-dioxan-2-yl)-ethyl]-1,3-dioxan;
trans-2-(4-Fluorphenyl)-5-[2-(trans-5-pentyl-1,3-dioxan-2-yl)-ethyl]-1,3-dioxan;
(E)-trans-2-(4-Fluorphenyl)-5-[2-(trans-5-propenyl-1,3-dioxan-2-yl)-ethyl]-1,3-dioxan;
(E)-trans-2-(4-Fluorphenyl)-5-[2-(trans-5-but-1-enyl-1,3-dioxan-2-yl)-ethyl]-1,3-dioxan;
trans-(4-Chlorphenyl)-5-[2-(trans-5-propyl-1,3-dioxan-2-yl)-ethyl]-1,3-dioxan;
trans-(4-Chlorphenyl)-5-[2-(trans-5-butyl-1,3-dioxan-2-yl)-ethyl]-1,3-dioxan;
trans-(4-Chlorphenyl)-5-[2-(trans-5-pentyl-1,3-dioxan-2-yl)-ethyl]-1,3-dioxan;
(E)-trans-(4-Chlorphenyl)-5-[2-(trans-5-propenyl-1,3-dioxan-2-yl)-ethyl]-1,3-dioxan;
(E)-trans-(4-Chlorphenyl)-5-[2-(trans-5-but-1-enyl-1,3-dioxan-2-yl)-ethyl]-1,3-dioxan;
(E)-trans-(4-Chlorphenyl)-5-[2-(trans-5-pent-1-enyl-1,3-dioxan-2-yl)-ethyl]-1,3-dioxan;
trans-2-(4-Trifluormethyl-phenyl)-5-[2-(trans-5-propyl-1,3-dioxan-2-yl)-ethyl]-1,3-dioxan, Smp. (C-S_{B}) 124°C, Klp. (S_{B}-I) 147°C;
trans-2-(4-Trifluormethyl-phenyl)-5-[2-(trans-5-butyl-1,3-dioxan-2-yl)-ethyl]-1,3-dioxan, Smp. (C-SB) 118,5°C, Klp. (S_{B}-I) 146°C;
trans-2-(4-Trifluormethyl-phenyl)-5-[2-(trans-5-pentyl-1,3-dioxan-2-yl)-ethyl)-1,3-dioxan;
(E)-trans-2-(4-Trifluormethyl-phenyl)-5-[2-(trans-5-propenyl-1,3-dioxan-2-yl)-ethyl]-1,3-dioxan, Smp. (C-S_{B}) 148°C, Klp. (S_{B}-I) 156,5°C;
(E)-trans-2-(4-Trifluormethyl-phenyl)-5-[2-(trans-5-but-1-enyl-1,3-dioxan-2-yl)-ethyl]-1,3-dioxan, Smp. (C-S_{B}) 150°C, Klp. (S_{B}-I) 161°C;
(E)-trans-2-(4-Trifluormethyl-phenyl)-5-[2-(trans-5-pent-1-enyl-1,3-dioxan-2-yl)-ethyl]-1,3-dioxan;
trans-2-(4-Difluormethoxy-phenyl)-5-[2-(trans-5-propyl-1,3-dioxan-2-yl)-ethyl]-1,3-dioxan;
trans-2-(4-Difluormethoxy-phenyl)-5-[2-(trans-5-butyl-1,3-dioxan-2-yl)-ethyl]-1,3-dioxan;
trans-2-(4-Difluormethoxy-phenyl)-5-[2-(trans-5-pentyl-1,3-dioxan-2-yl)-ethyl]-1,3-dioxan;
(E)-trans-2-(4-Difluormethoxy-phenyl)-5-[2-(trans-5-propenyl-1,3-dioxan-2-yl)-ethyl]-1,3-dioxan;
trans-2-(4-Trifluormethoxy-phenyl)-5-[2-(trans-5-propyl-1,3-dioxan-2-yl)-ethyl]-1,3-dioxan;
trans-2-(4-Trifluormethoxy-phenyl)-5-[2-(trans-5-butyl-1,3-dioxan-2-yl)-ethyl]-1,3-dioxan;
trans-2-(4-Trifluormethoxy-phenyl)-5-[2-(trans-5-pentyl-1,3-dioxan-2-yl)-ethyl]-1,3-dioxan;
(E)-trans-2-(4-Trifluormethoxy-phenyl)-5-[2-(trans-5-propenyl-1,3-dioxan-2-yl)-ethyl]-1,3-dioxan;
(E)-trans-2-(4-Trifluormethoxy-phenyl)-5-[2-(trans-5-but-1-enyl-1,3-dioxan-2-yl)-ethyl]-1,3-dioxan;
(E)-trans-2-(4-Trifluormethoxy-phenyl)-5-[2-(trans-5-pent-1-enyl-1,3-dioxan-2-yl)-ethyl]-1,3-dioxan;
trans-2-(4-Trifluormethoxy-phenyl)-5-[2-(trans-5-but-3-enyl-1,3-dioxan-2-yl)-ethyl]-1,3-dioxan;
trans-2-(4-Trifluormethoxy-phenyl)-5-[2-(trans-5-pent-4-enyl-1,3-dioxan-2-yl)-ethyl]-1,3-dioxan;
trans-2-(4-Trifluormethoxy-phenyl)-5-[2-[trans-5-(3-methoxypropyl)-1,3-dioxan-2-yl]-ethyl]-1,3-dioxan;
trans-2-[4-(2,2,2-Trifluorethoxy)-phenyl]-5-[2-(trans-5-propyl-1,3-dioxan-2-yl)-ethyl]-1,3-dioxan;
trans-2-[4-(2,2,2-Trifluorethoxy)-phenyl]-5-[2-(trans-5-butyl-1,3-dioxan-2-yl)-ethyl]-1,3-dioxan;
trans-2-[4-(2,2,2-Trifluorethoxy)-phenyl]-5-[2-(trans-5-pentyl-1,3-dioxan-2-yl)-ethyl]-1,3-dioxan;
(E)-trans-2-[4-(2,2,2-Trifluorethoxy)-phenyl]-5-[2-(trans-5-propenyl-1,3-dioxan-2-yl)-ethyl]-1,3-dioxan;
(E)-trans-2-[4-(2,2,2-Trifluorethoxy)-phenyl]-5-[2-(trans-5-but-1-enyl-1,3-dioxan-2-yl)-ethyl]-1,3-dioxan;
trans-2-(4-Tolyl)-5-[2-(trans-5-propyl-1,3-dioxan-2-yl)-ethyl]-1,3-dioxan;
trans-2-(4-Tolyl)-5-[2-(trans-5-pentyl-1,3-dioxan-2-yl)-ethyl]-1,3-dioxan;
trans-(4-Propyl-phenyl)-5-[2-(trans-5-propyl-1,3-dioxan-2-yl)-ethyl]-1,3-dioxan;
trans-(4-Propyl-phenyl)-5-[2-(trans-5-pentyl-1,3-dioxan-2-yl)-ethyl]-1,3-dioxan;
trans-(4-Ethoxy-phenyl)-5-[2-(trans-5-propyl-1,3-dioxan-2-yl)-ethyl]-1,3-dioxan;
trans-2-(4-Ethoxy-phenyl)-5-[2-(trans-5-pentyl-1,3-dioxan-2-yl)-ethyl]-1,3-dioxan;
trans-2-(3,4-Difluor-phenyl)-5-[2-(trans-5-propyl-1,3-dioxan-2-yl)-ethyl]-1,3-dioxan, Smp. (C-I) 82,9°C, S_{B}-N 76,4°C, Klp. (N-I) 76,5°C;
trans-2-(3,4-Difluor-phenyl)-5-[2-(trans-5-butyl-1,3-dioxan-2-yl)-ethyl]-1,3-dioxan, Smp. (C-I) 87,5°C, Klp. (S_{B}-I) 78,7°C;
trans-2-(3,4-Difluor-phenyl)-5-[2-(trans-5-pentyl-1,3-dioxan-2-yl)-ethyl]-1,3-dioxan;
trans-2-(3,4-Difluor-phenyl)-5-[2-(trans-5-hexyl-1,3-dioxan-2-yl)-ethyl]-1,3-dioxan;
(E)-trans-2-(3,4-Difluor-phenyl)-5-[2-(trans-5-propenyl-1,3-dioxan-2-yl)-ethyl]-1,3-dioxan, Smp. (C-I) 106,1°C, S_{B}-N 86,4°C, Klp. (N-I) 97,3°C;
(E)-trans-2-(3,4-Difluor-phenyl)-5-[2-(trans-5-but-1-enyl-1,3-dioxan-2-yl)-ethyl]-1,3-dioxan, Smp. (C-I) 97.1°C, Klp. (S_{B}-I) 94.8°C;
(E)-trans-2-(3,4-Difluor-phenyl)-5-[2-(trans-5-pent-1-enyl-1,3-dioxan-2-yl)-ethyl]-1,3-dioxan;
trans-2-(3,4-Difluor-phenyl)-5-[2-(trans-5-but-3-enyl-1,3-dioxan-2-yl)-ethyl]-1,3-dioxan;
trans-2-(3,4-Difluor-phenyl)-5-[2-[trans-5-(3-methoxy-propyl)-1,3-dioxan-2-yl]-ethyl]-1,3-dioxan;
trans-2-(4-Chlor-3-fluor-phenyl)-5-[2-(trans-5-propyl-1,3-dioxan-2-yl)-ethyl]-1,3-dioxan;
trans-2-(4-Chlor-3-fluor-phenyl)-5-[2-(trans-5-pentyl-1,3-dioxan-2-yl)-ethyl]-1,3-dioxan;
(E)-trans-2-(4-Chlor-3-fluor-phenyl)-5-[2-(trans-5-propenyl-1,3-dioxan-2-yl)-ethyl]-1,3-dioxan;
trans-2-(4-Difluormethoxy-3-fluor-phenyl)-5-[2-(trans-5-propyl-1,3-dioxan-2-yl)-ethyl]-1,3-dioxan;
trans-2-(4-Difluormethoxy-3-fluor-phenyl)-5-[2-(trans-5-butyl-1,3-dioxan-2-yl)-ethyl]-1,3-dioxan;
trans-2-(4-Difluormethoxy-3-fluor-phenyl)-5-[2-(trans-5-pentyl-1,3-dioxan-2-yl)-ethyl]-1,3-dioxan;
(E)-trans-2-(4-Difluormethoxy-3-fluor-phenyl)-5-[2-(trans-5-propenyl-1,3-dioxan-2-yl)-ethyl]-1,3-dioxan;
(E)-trans-2-(4-Difluormethoxy-3-fluor-phenyl)-5-[2-(trans-5-pent-1-enyl-1,3-dioxan-2-yl)-ethyl]-1,3-dioxan;
trans-2-(3-Fluor-4-trifluormethoxy-phenyl)-5-[2-(trans-5-propyl-1,3-dioxan-2-yl)-ethyl]-1,3-dioxan;
trans-2-(3-Fluor-4-trifluormethoxy-phenyl)-5-[2-(trans-5-butyl-1,3-dioxan-2-yl)-ethyl]-1,3-dioxan;
trans-2-(3-Fluor-4-trifluormethoxy-phenyl)-5-[2-(trans-5-pentyl-1,3-dioxan-2-yl)-ethyl]-1,3-dioxan;
(E)-trans-2-(3-Fluor-4-trifluormethoxy-phenyl)-5-[2-(trans-5-propenyl-1,3-dioxan-2-yl)-ethyl]-1,3-dioxan;
(E)-trans-2-(3-Fluor-4-trifluormethoxy-phenyl)-5-[2-(trans-5-but-1-enyl-1,3-dioxan-2-yl)-ethyl]-1,3-dioxan;
(E)-trans-2-(3-Fluor-4-trifluormethoxy-phenyl)-5-[2-(trans-5-pent-1-enyl-1,3-dioxan-2-yl)-ethyl]-1,3-dioxan;
trans-2-[3-Fluor-4-(2,2,2-trifluorethoxy-phenyl)-5-[2-(trans-5-propyl-1,3-dioxan-2-yl)-ethyl]-1,3-dioxan;
trans-2-[3-Fluor-4-(2,2,2-trifluorethoxy-phenyl)-5-[2-(trans-5-butyl-1,3-dioxan-2-yl)-ethyl]-1,3-dioxan;
trans-2-[3-Fluor-4-(2,2,2-trifluorethoxy-phenyl)-5-[2-(trans-5-pentyl-1,3-dioxan-2-yl)-ethyl]-1,3-dioxan;
trans-2-(3,4,5-Trifluor-phenyl)-5-[2-(trans-5-propyl-1,3-dioxan-2-yl)-ethyl]-1,3-dioxan, Smp. (C-I) 88,5°C;
trans-2-(3,4,5-Trifluor-phenyl)-5-[2-(trans-5-butyl-1,3-dioxan-2-yl)-ethyl]-1,3-dioxan;
trans-2-(3,4,5-Trifluor-phenyl)-5-[2-(trans-5-pentyl-1,3-dioxan-2-yl)-ethyl]-1,3-dioxan;
trans-2-(3,4,5-Trifluor-phenyl)-5-[2-(trans-5-hexyl-1,3-dioxan-2-yl)-ethyl]-1,3-dioxan;
(E)-trans-2-(3,4,5-Trifluor-phenyl)-5-[2-(trans-5-propenyl-1,3-dioxan-2-yl)-ethyl]-1,3-dioxan, Smp. (C-I) 112,7°C, Klp. (N-I) 63,6°C;
(E)-trans-2-(3,4,5-Trifluor-phenyl)-5-[2-(trans-5-but-1-enyl-1,3-dioxan-2-yl)-ethyl]-1,3-dioxan;
(E)-trans-2-(3,4,5-Trifluor-phenyl)-5-[2-(trans-5-pent-1-enyl-1,3-dioxan-2-yl)-ethyl]-1,3-dioxan;
trans-2-(3,4,5-Trifluor-phenyl)-5-[2-(trans-5-but-3-enyl-1,3-dioxan-2-yl)-ethyl]-1,3-dioxan;
trans-2-(3,4,5-Trifluor-phenyl)-5-[2-(trans-5-pent-4-enyl-1,3-dioxan-2-yl)-ethyl]-1,3-dioxan;
trans-2-(4-Difluormethoxy-3,5-difluor-phenyl)-5-[2-(trans-5-propyl-1,3-dioxan-2-yl)-ethyl]-1,3-dioxan;
trans-2-(4-Difluormethoxy-3,5-difluor-phenyl)-5-[2-(trans-5-butyl-1,3-dioxan-2-yl)-ethyl]-1,3-dioxan;
trans-2-(4-Difluormethoxy-3,5-difluor-phenyl)-5-[2-(trans-5-pentyl-1,3-dioxan-2-yl)-ethyl]-1,3-dioxan;
(E)-trans-2-(4-Difluormethoxy-3,5-difluor-phenyl)-5-[2-(trans-5-propenyl-1,3-dioxan-2-yl)-ethyl]-1,3-dioxan;
(E)-trans-2-(4-Difluormethoxy-3,5-difluor-phenyl)-5-[2-(trans-5-but-1-enyl-1,3-dioxan-2-yl)-ethyl]-1,3-dioxan;
(E)-trans-2-(4-Difluormethoxy-3,5-difluor-phenyl)-5-[2-(trans-5-pent-1-enyl-1,3-dioxan-2-yl)-ethyl]-1,3-dioxan;
trans-2-[3,5-Difluor-4-(2,2,2-trifluorethoxy)-phenyl]-5-[2-(trans-5-propyl-1,3-dioxan-2-yl)-ethyl]-1,3-dioxan;
trans-2-[3,5-Difluor-4-(2,2,2-trifluorethoxy)-phenyl]-5-[2-(trans-5-butyl-1,3-dioxan-2-yl)-ethyl]-1,3-dioxan;
trans-2-[3,5-Difluor-4-(2,2,2-trifluorethoxy)-phenyl]-5-[2-(trans-5-pentyl-1,3-dioxan-2-yl)-ethyl]-1,3-dioxan;
(E)-trans-2-[3,5-Difluor-4-(2,2,2-trifluorethoxy)-phenyl]-5-[2-(trans-5-propenyl-1,3-dioxan-2-yl)-ethyl]-1,3-dioxan;
(E)-trans-2-[3,5-Difluor-4-(2,2,2-trifluorethoxy)-phenyl]-5-[2-(trans-5-but-1-enyl-1,3-dioxan-2-yl)-ethyl]-1,3-dioxan;
(E)-trans-2-[3,5-Difluor-4-(2,2,2-trifluorethoxy)-phenyl]-5-[2-(trans-5-pent-1-enyl-1,3-dioxan-2-yl)-ethyl]-1,3-dioxan;
4-[trans-5-[2-(trans-5-Butyl-1,3-dioxan-2-yl)-ethyl]-1,3-dioxan-2-yl]-phenyl-isothiocyanat;
4-[trans-5-[2-(trans-5-Propyl-1,3-dioxan-2-yl)-ethyl]-1,3-dioxan-2-yl]-benzonitril;
4-[trans-5-[2-(trans-5-Butyl-1,3-dioxan-2-yl)-ethyl]-1,3-dioxan-2-yl]-benzonitril;
4-[trans-5-[2-(trans-5-Pentyl-1,3-dioxan-2-yl)-ethyl]-1,3-dioxan-2-yl]-benzonitril;
(E)-4-[trans-5-[2-(trans-5-Propenyl-1,3-dioxan-2-yl)-ethyl]-1,3-dioxan-2-yl]-benzonitril;
(E)-4-[trans-5-[2-(trans-5-But-1-enyl-1,3-dioxan-2-yl)-ethyl]-1,3-dioxan-2-yl]-benzonitril;
trans-2-(2,3-Difluor-4-propyl-phenyl)-5-[2-(trans-5-butyl-1,3-dioxan-2-yl)-ethyl]-1,3-dioxan;
trans-2-(2,3-Difluor-4-pentyl-phenyl)-5-[2-(trans-5-butyl-1,3-dioxan-2-yl)-ethyl]-1,3-dioxan;
trans-2-(2,3-Difluor-4-ethoxy-phenyl)-5-[2-(trans-5-butyl-1,3-dioxan-2-yl)-ethyl]-1,3-dioxan;
trans-2-(2,3-Difluor-4-butoxy-phenyl)-5-[2-(trans-5-butyl-1,3-dioxan-2-yl)-ethyl]-1,3-dioxan;
trans-2-(6-Butyl-2-naphthyl)-5-[2-(trans-5-butyl-1,3-dioxan-2-yl)-ethyl]-1,3-dioxan;
trans-2-(6-Ethoxy-2-naphthyl)-5-[2-(trans-5-butyl-1,3-dioxan-2-yl)-ethyl]-1,3-dioxan;
trans-2-(6-Butoxy-2-naphthyl)-5-[2-(trans-5-butyl-1,3-dioxan-2-yl)-ethyl]-1,3-dioxan;
trans-2-(2-Pentyl-5-pyridyl)-5-[2-(trans-5-butyl-1,3-dioxan-2-yl)-ethyl]-1,3-dioxan;
trans-2-(2-Butyl-5-pyrimidyl)-5-[2-(trans-5-butyl-1,3-dioxan-2-yl)-ethyl]-1,3-dioxan;
trans-2-(4-Fluor-3-cyclohexenyl)-5-[2-(trans-5-propyl-1,3-dioxan-2-yl)-ethyl]-1,3-dioxan;
trans-2-(4-Fluor-3-cyclohexenyl)-5-[2-(trans-5-butyl-1,3-dioxan-2-yl)-ethyl]-1,3-dioxan;
trans-2-(4-Fluor-3-cyclohexenyl)-5-[2-(trans-5-pentyl-1,3-dioxan-2-yl)-ethyl]-1,3-dioxan;
(E)-trans-2-(4-Fluor-3-cyclohexenyl)-5-[2-(trans-5-propenyl-1,3-dioxan-2-yl)-ethyl]-1,3-dioxan;
(E)-trans-2-(4-Fluor-3-cyclohexenyl)-5-[2-(trans-5-but-1-enyl-1,3-dioxan-2-yl)-ethyl]-1,3-dioxan;
trans-2-(4-Trifluormethyl-3-cyclohexenyl)-5-[2-(trans-5-butyl-1,3-dioxan-2-yl)-ethyl]-1,3-dioxan.

### Beispiel 2

Eine Lösung aus 1.72 g (E)-2-[4-(5-But-1-enyl-1,3-dioxan-2-yl)butyl]-1,3-propandiol und 1,0 g 3,4-Difluorbenzaldehyd in 30 ml Benzol wird mit 35 mg p-Toluolsulfonsäure-monohydrat 1 Stunde zu leichtem Sieden erhitzt. Nach Neutralisation der Lösung mit einigen Tropfen Triethylamin wird mit Wasser gewaschen, über Natriumsulfat getrocknet, filtriert und das Filtrat eingedampft. Das Rohprodukt wird an 40 g Kieselgel mit Hexan/Ethylacetat 5 % (v/v) chromatographiert. Zweifache Kristallisation des erhaltenen Isomerengemisches aus Hexan liefert reines (E)-trans-2-(3,4-Difluorphenyl)-5-[4-(trans-5-but-1-enyl-1,3-dioxan-2-yl)-ethyl]-1,3-dioxan.

Das als Ausgangsmaterial verwendete (E)-2-[4-(5-But-1-enyl-1,3-dioxan-2-yl)-butyl]-1,3-propandiol wird in analoger Weise zu dem in Beispiel 1 beschriebenen Ethyl-Derivat ausgehend von 2-(4-Brombutyl)-dioxolan hergestellt:

In analoger Weise können folgende Verbindungen hergestellt werden:
trans-2-(3,4-Difluor-phenyl)-5-[4-(trans-5-butyl-1,3-dioxan-2-yl)-butyl]-1,3-dioxan;
trans-2-(3,4,5-Trifluor-phenyl)-5-[4-(trans-5-butyl-1,3-dioxan-2-yl)-butyl]-1,3-dioxan;
trans-2-(-4-Trifluormethoxy-phenyl)-5-[4-(trans-5-butyl-1,3-dioxan-2-yl)-butyl]-1,3-dioxan;
trans-2-(4-Difluormethoxy-3-fluor-phenyl)-5-[4-(trans-5-butyl-1,3-dioxan-2-yl)-butyl]-1,3-dioxan;
trans-2-(4-Difluormethoxy-3,5-difluor-phenyl)-5-[4-(trans-5-butyl-1,3-dioxan-2-yl)-butyl]-1,3-dioxan;
trans-2-[4-(2,2,2-Trifluorethoxy)-phenyl]-5-[4-(trans-5-butyl-1,3-dioxan-2-yl)-butyl]-1,3-dioxan;
trans-2-[3-Fluor-4-(2,2,2-Trifluorethoxy)-phenyl]-5-[4-(trans-5-butyl-1,3-dioxan-2-yl)-butyl]-1,3-dioxan;
trans-2-[3,5-Difluor-4-(2,2,2-Trifluorethoxy)-phenyl]-5-[4-(trans-5-butyl-1,3-dioxan-2-yl)-butyl]-1,3-dioxan;
trans-2-(4-Chlor-3-fluor-phenyl)-5-[4-(trans-5-butyl-1,3-dioxan-2-yl)-butyl]-1,3-dioxan;
trans-2-(4-Cyanophenyl)-5-[4-(trans-5-butyl-1,3-dioxan-2-yl)-butyl]-1,3-dioxan;
trans-2-(trans-4-Pentylcyclohexyl)-5-[4-(trans-5-butyl-1,3-dioxan-2-yl)-butyl]-1,3-dioxan;
trans-2-Pentyl-5-[4-(trans-5-butyl-1,3-dioxan-2-yl)-butyl]-1,3-dioxan;
trans-2-Butyl-5-[4-(trans-5-butyl-1,3-dioxan-2-yl)-butyl]-1,3-dioxan.

### Beispiel 3

Eine Lösung aus 1,2 g 2-(5-Butyl-1,3-dioxan-2-yl)-1,3-propandiol und 2,4 ml Capronaldehyd wurde mit 14 mg p-Toluolsulfonsäure-monohydrat versetzt und 45 Minuten bei 70°C gerührt. Das Reaktionsgemisch wurde mit einigen Tropfen Triethylamin neutralisiert und nach dem Erkalten mit gesättigter Kochsalzlösung gewaschen, über Natriumsulfat getrocknet, filtriert und das Filtrat eingeengt. Das Rohprodukt (1,92 g) wurde an 50 g Kieselgel mit Hexan/Ethylacetat 5% (v/v) chromatographiert. Es resultierten 1,52 g Isomerengemisch mit einem trans/trans-Anteil (GC) von 44%. Zweimalige Umkristallisation aus Hexan ergab 0,42 g reines trans-5-Butyl-trans-2'-pentyl-2,5'-bi-1,3-dioxan, Smp. (C-S_{B}) 93,3°C, Klp. (Sg-I) 127,0°C.

Das als Ausgangsmaterial verwendete 2-(5-Butyl-1,3-dioxan-2-yl)-1,3-propandiol wurde wie folgt hergestellt:
a) Ein Gemisch aus 17,45 g 2-Butyl-1,3-propandiol, 26,4 g (Ethoxymethyliden)-malonsäure-diethylester, 250 ml Toluol und 0,285 g p-Toluolsulfonsäure-monohydrat wurde 1 Stunde zum Sieden erhitzt, wobei feuchtes Toluol abdestilliert und durch Zugabe von frischem Toluol ersetzt wurde. Das Reaktionsgemisch wurde mit Triethylamin neutralisiert und nach dem Erkalten mit gesättigter Kochsalzlösung gewaschen, über Natriumsulfat getrocknet und eingeengt. Das Rohprodukt (42,35 g) wurde an 520 g Kieselgel mit Hexan/Ethylacetat (4:1) chromatographiert. Man erhielt 33,0 g öligen (5-Butyl-1,3-dioxan-2-yl)-malonsäure-diethylester.
b) Eine Lösung von 33,0 g (5-Butyl-1,3-dioxan-2-yl)-malonsäure-diethylester in 200 ml Tetrahydrofuran wurde innert 2 Stunden bei 3-5°C in einer Suspension von 8,35g Lithiumaluminiumhydrid in 300 ml Tetrahydrofuran getropft. Das Reaktionsgemisch wurde noch 15 Minuten bei 5°C und 2 Stunden bei Raumtemperatur gerührt. Nach dem Erkalten wurde vorsichtig 3N Natronlauge zugetropft, bis sich ein weisser Klumpen absetzte. Die Lösung wurde dekantiert und der Rückstand mehrmals mit Diethylether gewaschen. Die vereinigten organischen Phasen wurden mit 40 ml konz. Natriumbicarbonat-Lösung und fünfmal je 40 ml konz. Kochsalzlösung gewaschen, über Natriumsulfat getrocknet, filtriert und eingeengt. Das Rohprodukt (22,8 g) wurde ohne weitere Reinigung in der nächsten Stufe eingesetzt; es enthielt nach GC 93% 2-(5-Butyl-1,3-dioxan-2-yl)-1,3-propandiol (trans/cis-Verhältnis 2,3:1).

In analoger Weise können hergestellt werden:
trans,trans-5,2'-Dipropyl-2,5'-bi-1,3-dioxan, Smp. (C-S_{B}) 85,4°C, Klp. (S_{B}-I) 125,5°C
trans-5-Propyl-trans-2'-butyl-2,5'-bi-1,3-dioxan;
trans-5-Propyl-trans-2'-pentyl-2,5'-bi-1,3-dioxan;
trans-5-Butyl-trans-2'-propyl-2,5'-bi-1,3-dioxan;
trans,trans-5,2'-Dibutyl-2,5'-bi-1,3-dioxan;
trans-5-Pentyl-trans-2'-propyl-2,5'-bi-1,3-dioxan;
trans-5-Pentyl-trans-2'-butyl-2,5'-bi-1,3-dioxan;
trans,trans-5,2'-Dipentyl-2,5'-bi-1,3-dioxan, Smp. (C-S_{B}) 92,8°C, Klp. (S_{B}-I) 134,0°C
(E)-trans-5-Propenyl-trans-2'-propyl-2,5'-bi-1,3-dioxan;
(E)-trans-5-Propenyl-trans-2'-butyl-2,5'-bi-1,3-dioxan;
(E)-trans-5-Propenyl-trans-2'-pentyl-2,5'-bi-1,3-dioxan;
(E)-trans-5-But-1-enyl-trans-2'-propyl-2,5'-bi-1,3-dioxan;
(E)-trans-5-But-1-enyl-trans-2'-butyl-2,5'-bi-1,3-dioxan;
(E)-trans-5-But-1-enyl-trans-2'-pentyl-2,5'-bi-1,3-dioxan;
(E)-trans-5-Pent-1-enyl-trans-2-propyl-2,5'-bi-1,3-dioxan;
(E)-trans-5-Pent-1-enyl-trans-2-butyl-2,5'-bi-1,3-dioxan;
(E)-trans-5-Pent-1-enyl-trans-2-pentyl-2,5'-bi-1,3-dioxan;
(E)-trans-5-But-3-enyl-trans-2-butyl-2,5'-bi-1,3-dioxan;
trans-5-Propyl-trans-2'-(trans-4-propylcyclohexyl)-2,5'-bi-1,3-dioxan;
trans-5-Propyl-trans-2'-(trans-4-butylcyclohexyl)-2,5'-bi-1,3-dioxan;
trans-5-Propyl-trans-2'-(trans-4-pentylcyclohexyl)-2,5'-bi-1,3-dioxan;
trans-5-Butyl-trans-2'-(trans-4-propylcyclohexyl)-2,5'-bi-1,3-dioxan;
trans-5-Butyl-trans-2'-(trans-4-butylcyclohexyl)-2,5'-bi-1,3-dioxan;
trans-5-Butyl-trans-2'-(trans-4-pentylcyclohexyl)-2,5'-bi-1,3-dioxan;
trans-5-Pentyl-trans-2'-(trans-4-propylcyclohexyl)-2,5'-bi-1,3-dioxan;
trans-5-Pentyl-trans-2'-(trans-4-butylcyclohexyl)-2,5'-bi-1,3-dioxan;
trans-5-Pentyl-trans-2'-(trans-4-pentylcyclohexyl)-2,5'-bi-1,3-dioxan;
(E)-trans-5-But-1-enyl-trans-2'-(trans-4-butylcyclohexyl)-2,5'-bi-1,3-dioxan;
trans-5-Propyl-trans-2'-(4-fluorphenyl)-2,5'-bi-1,3-dioxan;
trans-5-Butyl-trans-2'-(4-fluorphenyl)-2,5'-bi-1,3-dioxan;
trans-5-Pentyl-trans-2'-(4-fluorphenyl)-2,5'-bi-1,3-dioxan;
(E)-trans-5-Propenyl-trans-2'-(4-fluorphenyl)-2,5'-bi-1,3-dioxan;
(E)-trans-5-But-1-enyl-trans-2'-(4-fluorphenyl)-2,5'-bi-1,3-dioxan;
trans-5-Butyl-trans-2'-(4-chlorphenyl)-2,5'-bi-1,3-dioxan;
trans-5-Pentyl-trans-2'-(4-chlorphenyl)-2,5'-bi-1,3-dioxan;
trans-5-Propyl-trans-2'-(4-trifluormethyl-phenyl)-2,5'-bi-1,3-dioxan, Smp. (C-S_{B}) 143,4°C, Klp. (S_{B}-I) 188,5°C;
trans-5-Butyl-trans-2'-(4-trifluormethyl-phenyl)-2,5'-bi-1,3-dioxan, Smp. (C-S_{B}) 115,0°C, Klp. (S_{B}-I) 174,5°C;
trans-5-Pentyl-trans-2'-(4-trifluormethyl-phenyl)-2,5'-bi-1,3-dioxan, Smp. (C-S_{B}) 101,1°C, Klp. (S_{B}-I) 184,0°C;
(E)-trans-5-Propenyl-trans-2'-(4-trifluormethyl-phenyl)-2,5'-bi-1,3-dioxan;
(E)-trans-5-But-1-enyl-trans-2'-(4-trifluormethyl-phenyl)-2,5'-bi-1,3-dioxan;
(E)-trans-5-Pent-1-enyl-trans-2'-(4-trifluormethyl-phenyl)-2,5'-bi-1,3-dioxan;
trans-5-Propyl-trans-2'-(4-trifluormethoxy-phenyl)-2,5'-bi-1,3-dioxan, Smp. (C-S_{B}) 125,2°C, Klp. (S_{B}-I) 195,8°C;
trans-5-Butyl-trans-2'-(4-trifluormethoxy-phenyl)-2,5'-bi-1,3-dioxan, Smp. (C-S_{B}) 102,7°C, Klp. (S_{B}-I) 194,3°C;
trans-5-Pentyl-trans-2'-(4-trifluormethoxy-phenyl)-2,5'-bi-1,3-dioxan, Smp. (C-S_{B}) 108°C, Klp. (S_{B}-I) 192°C;
(E)-trans-5-But-1-enyl-trans-2'-(4-trifluormethoxy-phenyl)-2,5'-bi-1,3-dioxan;
trans-5-Propyl-trans-2'-(4-difluormethoxy-phenyl)-2,5'-bi-1,3-dioxan;
trans-5-Butyl-trans-2'-(4-difluormethoxy-phenyl)-2,5'-bi-1,3-dioxan;
trans-5-Pentyl-trans-2'-(4-difluormethoxy-phenyl)-2,5'-bi-1,3-dioxan;
trans-5-Butyl-trans-2'-[4-(2,2,2-trifluorethoxy)-phenyl]-2,5'-bi-1,3-dioxan;
trans-5-Pentyl-trans-2'-[4-(2,2,2-trifluorethoxy)-phenyl]-2,5'-bi-1,3-dioxan; trans-5-Butyl-trans-2'-(4-tolyl)-2,5'-bi-1,3-dioxan;
trans-5-Pentyl-trans-2'-(4-tolyl)-2,5'-bi-1,3-dioxan;
trans-5-Propyl-trans-2'-(4-propylphenyl)-2,5'-bi-1,3-dioxan;
trans-5-Butyl-trans-2'-(4-propylphenyl)-2,5'-bi-1,3-dioxan;
trans-5-Pentyl-trans-2'-(4-propylphenyl)-2,5'-bi-1,3-dioxan;
trans-5-Propyl-trans-2'-(4-ethoxy-phenyl)-2,5'-bi-1,3-dioxan;
trans-5-Pentyl-trans-2'-(4-ethoxy-phenyl)-2,5'-bi-1,3-dioxan;
trans-5-Propyl-trans-2'-(3,4-difluor-phenyl)-2,5'-bi-1,3-dioxan, Smp. (C-S_{B}) 127,7°C, Klp. (S_{B}-I) 139,5°C;
trans-5-Butyl-trans-2'-(3,4-difluor-phenyl)-2,5'-bi-1,3-dioxan, Smp. (C-S_{B}) 102,0°C, Klp. (S_{B}-I) 132,1°C;
trans-5-Pentyl-trans-2'-(3,4-difluor-phenyl)-2,5'-bi-1,3-dioxan, Smp. (C-S_{B}) 95,5°C, S_{B}-S₂ 116,5, S₂-S_{A} 121,8, Klp. (S_{A}-I) 124,8°C;
(E)-trans-5-Propenyl-trans-2'-(3,4-difluor-phenyl)-2,5'-bi-1,3-dioxan;
(E)-trans-5-But-1-enyl-trans-2'-(3,4-difluor-phenyl)-2,5'-bi-1,3-dioxan;
(E)-trans-5-Pent-1-enyl-trans-2'-(3,4-difluor-phenyl)-2,5'-bi-1,3-dioxan;
trans-5-But-3-enyl-trans-2'-(3,4-difluor-phenyl)-2,5'-bi-1,3-dioxan;
trans-5-Pent-4-enyl-trans-2'-(3,4-difluor-phenyl)-2,5'-bi-1,3-dioxan;
trans-5-Propyl-trans-2'-(4-chlor-3-fluor-phenyl)-2,5'-bi-1,3-dioxan;
trans-5-Butyl-trans-2'-(4-chlor-3-fluor-phenyl)-2,5'-bi-1,3-dioxan;
trans-5-Pentyl-trans-2'-(4-chlor-3-fluor-phenyl)-2,5'-bi-1,3-dioxan;
trans-5-Propyl-trans-2'-(4-difluormethoxy-3-fluor-phenyl)-2,5'-bi-1,3-dioxan;
trans-5-Butyl-trans-2'-(4-difluormethoxy-3-fluor-phenyl)-2,5'-bi-1,3-dioxan;
trans-5-Pentyl-trans-2'-(4-difluormethoxy-3-fluor-phenyl)-2,5'-bi-1,3-dioxan;
(E)-trans-5-Propenyl-trans-2'-(4-difluormethoxy-3-fluor-phenyl)-2,5'-bi-1,3-dioxan;
(E)-trans-5-But-1-enyl-trans-2'-(4-difluormethoxy-3-fluor-phenyl)-2,5'-bi-1,3-dioxan;
(E)-trans-5-Pent-1-enyl-trans-2'-(4-difluormethoxy-3-fluor-phenyl)-2,5'-bi-1,3-dioxan;
trans-5-Propyl-trans-2'-(3-fluor-4-trifluormethoxy-phenyl)-2,5'-bi-1,3-dioxan;
trans-5-Butyl-trans-2'-(3-fluor-4-trifluormethoxy-phenyl)-2,5'-bi-1,3-dioxan;
trans-5-Pentyl-trans-2'-(3-fluor-4-trifluormethoxy-phenyl)-2,5'-bi-1,3-dioxan;
trans-5-Propyl-trans-2-[3-fluor-4-(2,2,2-trifluorethoxy)-phenyl]-2,5'-bi-1,3-dioxan;
trans-5-Butyl-trans-2-[3-fluor-4-(2,2,2-trifluorethoxy)-phenyl]-2,5'-bi-1,3-dioxan;
trans-5-Pentyl-trans-2-[3-fluor-4-(2,2,2-trifluorethoxy)-phenyl]-2,5'-bi-1,3-dioxan;
trans-5-Propyl-trans-2'-(3,4,5-trifluor-phenyl)-2,5'-bi-1,3-dioxan, Smp. (C-I) 117,3°C;
trans-5-Butyl-trans-2'-(3,4,5-trifluor-phenyl)-2,5'-bi-1,3-dioxan, Smp. (C-I) 99,8°C, Klp. (S_{A}-I) 91,5°C;
trans-5-Pentyl-trans-2'-(3,4,5-trifluor-phenyl)-2,5'-bi-1,3-dioxan, Smp. (C-I) 93,8°C, Klp. (S_{A}-I) 92,0°C;
(E)-trans-5-Propenyl-trans-2'-(3,4,5-trifluor-phenyl)-2,5'-bi-1,3-dioxan;
(E)-trans-5-But-1-enyl-trans-2'-(3,4,5-trifluor-phenyl)-2,5'-bi-1,3-dioxan;
trans-5-Propyl-trans-2'-(4-difluormethoxy-3,5-difluor-phenyl)-2,5'-bi-1,3-dioxan;
trans-5-Butyl-trans-2'-(4-difluormethoxy-3,5-difluor-phenyl)-2,5'-bi-1,3-dioxan;
trans-5-Pentyl-trans-2'-(4-difluormethoxy-3,5-difluor-phenyl)-2,5'-bi-1,3-dioxan;
(E)-trans-5-Propenyl-trans-2'-(4-difluormethoxy-3,5-difluor-phenyl)-2,5'-bi-1,3-dioxan;
(E)-trans-5-But-1-enyl-trans-2'-(4-difluormethoxy-3,5-difluor-phenyl)-2,5'-bi-1,3-dioxan;
trans-5-Propyl-trans-2'-[3,5-difluor-4-(2,2,2-trifluorethoxy)-phenyl]-2,5'-bi-1,3-dioxan;
trans-5-Butyl-trans-2'-[3,5-difluor-4-(2,2,2-trifluorethoxy)-phenyl]-2,5'-bi-1,3-dioxan;
trans-5-Pentyl-trans-2'-[3,5-difluor-4-(2,2,2-trifluorethoxy)-phenyl]-2,5'-bi-1,3-dioxan;
(E)-trans-5-Propenyl-trans-2'-[3,5-difluor-4-(2,2,2-trifluorethoxy)-phenyl]-2,5'-bi-1,3-dioxan;
(E)-trans-5-But-1-enyl-trans-2'-[3,5-difluor-4-(2,2,2-trifluorethoxy)-phenyl]-2,5'-bi-1,3-dioxan;
4-(trans-5-Propyl-[2,5'-bi-1,3-dioxan]-trans-2'-yl)-benzonitril;
4-(trans-5-Butyl-[2,5'-bi-1,3-dioxan]-trans-2'-yl)-benzonitril;
4-(trans-5-Pentyl-[2,5'-bi-1,3-dioxan]-trans-2'-yl)-benzonitril;
4-(trans-5-Propyl-[2,5'-bi-1,3-dioxan]-trans-2'-yl)-2-fluor-benzonitril;
4-(trans-5-Butyl-[2,5'-bi-1,3-dioxan]-trans-2'-yl)-2-fluor-benzonitril;
4-(trans-5-Pentyl-[2,5'-bi-1,3-dioxan]-trans-2'-yl)-2-fluor-benzonitril;
trans-5-Propyl-trans-2'-(4-fluor-3-cyclohexenyl)-2,5'-bi-1,3-dioxan;
trans-5-Butyl-trans-2'-(4-fluor-3-cyclohexenyl)-2,5'-bi-1,3-dioxan, Smp. (C-S_{B}) 117.7°C, Klp. (S_{B}-I) 137.5°C;
trans-5-Pentyl-trans-2'-(4-fluor-3-cyclohexenyl)-2,5'-bi-1,3-dioxan;
(E)-trans-5-Propenyl-trans-2'-(4-fluor-3-cyclohexenyl)-2,5'-bi-1,3-dioxan;
(E)-trans-5-But-1-enyl-trans-2'-(4-fluor-3-cyclohexenyl)-2,5'-bi-1,3-dioxan;
trans-5-Propyl-trans-2'-(4-trifluormethyl-3-cyclohexenyl)-2,5'-bi-1,3-dioxan;
trans-5-Butyl-trans-2'-(4-trifluormethyl-3-cyclohexenyl)-2,5'-bi-1,3-dioxan;
trans-5-Pentyl-trans-2'-(4-trifluormethyl-3-cyclohexenyl)-2,5'-bi-1,3-dioxan.

### Beispiel 4

Ein Gemisch aus 921 mg 1,2-Difluor-4-(3-methoxyallyl)-benzol, 1133 mg 2-(5-Butyl-1,3-dioxan-2-yl)-1,3- propandiol, 25 ml Benzol und 14,8 mg p-Toluolsulfonsäure-monohydrat wurde 1 Stunde auf 74°C erhitzt, dann mit 5 Tropfen Triethylamin, neutralisiert und mit 40 ml Diethylether verdünnt. Die organische Phase wurde abgetrennt, zweimal mit je 10 ml Wasser gewaschen, über Natriumsulfat getrocknet, filtriert und das Filtrat eingeengt. Das gelbliche halbfeste Rohprodukt (1,82 g) wurde an 23 g Kieselgel mit Hexan/5% Ethylacetat chromatographiert. Zweimalige Umkristallisation des erhaltenen Isomerengemisches (1294 mg) aus Hexan lieferte 403 mg reines trans-5-Butyl-trans-2'-(3,4-difluor-phenethyl)-2,5'-bi-1,3-dioxan, Smp. (C-S_{B}) 89,9°C, Klp. (S_{B}-I) 110,4°C.

In analoger Weise können hergestellt werden:
trans-5-Propyl-trans-2'-(4-fluor-phenethyl)-2,5'-bi-1,3-dioxan;
trans-5-Butyl-trans-2'-(4-fluor-phenethyl)-2,5'-bi-1,3-dioxan;
trans-5-Pentyl-trans-2'-(4-fluor-phenethyl)-2,5'-bi-1,3-dioxan;
trans-5-Butyl-trans-2'-(4-chlor-phenethyl)-2,5'-bi-1,3-dioxan;
trans-5-Propyl-trans-2'-(4-trifluormethyl-phenethyl)-2,5'-bi-1,3-dioxan;
trans-5-Butyl-trans-2'-(4-trifluormethyl-phenethyl)-2,5'-bi-1,3-dioxan;
trans-5-Pentyl-trans-2'-(4-trifluormethyl-phenethyl)-2,5'-bi-1,3-dioxan;
trans-5-Propyl-trans-2'-(4-trifluormethoxy-phenethyl)-2,5'-bi-1,3-dioxan;
trans-5-Butyl-trans-2'-(4-trifluormethoxy-phenethyl)-2,5'-bi-1,3-dioxan, Smp. (C-S_{B}) 93,1°C, Klp. (S_{B}-I) 158,3°C;
trans-5-Pentyl-trans-2'-(4-trifluormethoxy-phenethyl)-2,5'-bi-1,3-dioxan;
(E)-trans-5-Propenyl-trans-2'-(4-trifluormethoxy-phenethyl)-2,5'-bi-1,3-dioxan;
(E)-trans-5-But-1-enyl-trans-2'-(4-trifluormethoxy-phenethyl)-2,5'-bi-1,3-dioxan;
trans-5-Propyl-trans-2'-(4-difluormethoxy-phenethyl)-2,5'-bi-1,3-dioxan;
trans-5-Butyl-trans-2'-(4-difluormethoxy-phenethyl)-2,5'-bi-1,3-dioxan;
trans-5-Pentyl-trans-2'-(4-difluormethoxy-phenethyl)-2,5'-bi-1,3-dioxan;
trans-5-Butyl-trans-2'-[4-(2,2,2-trifluorethoxy)-phenethyl]-2,5'-bi-1,3-dioxan;
trans-5-Propyl-trans-2'-(3,4-difluor-phenethyl)-2,5'-bi-1,3-dioxan, Smp. (C-S_{B}) 100,3°C, Klp. (S_{B}-I) 105,5°C;
trans-5-Pentyl-trans-2'-(3,4-difluor-phenethyl)-2,5'-bi-1,3-dioxan, Smp. (C-S_{B}) 96,0°C, Klp. (S_{B}-I) 112,0°C;
trans-5-Hexyl-trans-2'-(3,4-difluor-phenethyl)-2,5'-bi-1,3-dioxan;
(E)-trans-5-Propenyl-trans-2'-(3,4-difluor-phenethyl)-2,5'-bi-1,3-dioxan;
(E)-trans-5-But-1-enyl-trans-2'-(3,4-difluor-phenethyl)-2,5'-bi-1,3-dioxan;
(E)-trans-5-Pent-1-enyl-trans-2'-(3,4-difluor-phenethyl)-2,5'-bi-1,3-dioxan;
trans-5-But-3-enyl-trans-2'-(3,4-difluor-phenethyl)-2,5'-bi-1,3-dioxan;
trans-5-Pent-4-enyl-trans-2'-(3,4-difluor-phenethyl)-2,5'-bi-1,3-dioxan;
trans-5-(3-Methoxy-propyl)-trans-2'-(3,4-difluor-phenethyl)-2,5'-bi-1,3-dioxan;
trans-5-Propyl-trans-2'-(4-chlor-3-fluor-phenethyl)-2,5'-bi-1,3-dioxan;
trans-5-Butyl-trans-2'-(4-chlor-3-fluor-phenethyl)-2,5'-bi-1,3-dioxan;
trans-5-Pentyl-trans-2'-(4-chlor-3-fluor-phenethyl)-2,5'-bi-1,3-dioxan;
trans-5-Propenyl-trans-2'-(4-chlor-3-fluor-phenethyl)-2,5'-bi-1,3-dioxan;
trans-5-Propyl-trans-2'-(3-fluor-4-trifluormethoxy-phenethyl)-2,5'-bi-1,3-dioxan;
trans-5-Butyl-trans-2'-(3-fluor-4-trifluormethoxy-phenethyl)-2,5'-bi-1,3-dioxan;
trans-5-Propyl-trans-2'-(4-difluormethoxy-3-fluor-phenethyl)-2,5'-bi-1,3-dioxan;
trans-5-Butyl-trans-2'-(4-difluormethoxy-3-fluor-phenethyl)-2,5'-bi-1,3-dioxan;
trans-5-Pentyl-trans-2'-(4-difluormethoxy-3-fluor-phenethyl)-2,5'-bi-1,3-dioxan;
(E)-trans-5-Propenyl-trans-2'-(4-difluormethoxy-3-fluor-phenethyl)-2,5'-bi-1,3-dioxan;
(E)-trans-5-But-1-enyl-trans-2'-(4-difluormethoxy-3-fluor-phenethyl)-2,5'-bi-1,3-dioxan;
trans-5-Butyl-trans-2'-[3-fluor-4-(2,2,2-trifluorethoxy)-phenethyl]-2,5'-bi-1,3-dioxan;
trans-5-Butyl-trans-2'-(3-chlor-4-trifluormethyl-phenethyl)-2,5'-bi-1,3-dioxan;
trans-5-Propyl-trans-2'-(3,4,5-trifluor-phenethyl)-2,5'-bi-1,3-dioxan;
trans-5-Butyl-trans-2'-(3,4,5-trifluor-phenethyl)-2,5'-bi-1,3-dioxan;
trans-5-Pentyl-trans-2'-(3,4,5-trifluor-phenethyl)-2,5'-bi-1,3-dioxan;
(E)-trans-5-Propenyl-trans-2'-(3,4,5-trifluor-phenethyl)-2,5'-bi-1,3-dioxan;
(E)-trans-5-But-1-enyl-trans-2'-(3,4,5-trifluor-phenethyl)-2,5'-bi-1,3-dioxan;
trans-5-Propyl-trans-2'-(4-difluormethoxy-3,5-difluor-phenethyl)-2,5'-bi-1,3-dioxan, Smp. (C-I) 91,6°C, Klp. (S_{B}-I) 89°C;
trans-5-Butyl-trans-2'-(4-difluormethoxy-3,5-difluor-phenethyl)-2,5'-bi-1,3-dioxan, Smp. (C-S_{B}) 78,3°C, Klp. (S_{B}-I) 95,7°C;
trans-5-Pentyl-trans-2'-(4-difluormethoxy-3,5-difluor-phenethyl)-2,5'-bi-1,3-dioxan;
(E)-trans-5-Propenyl-trans-2'-(4-difluormethoxy-3,5-difluor-phenethyl)-2,5'-bi-1,3-dioxan;
(E)-trans-5-But-1-enyl-trans-2'-(4-difluormethoxy-3,5-difluor-phenethyl)-2,5'-bi-1,3-dioxan;
trans-5-Butyl-trans-2'-[3,5-difluor-4-(2,2,2-trifluorethoxy)-phenethyl]-2,5'-bi-1,3-dioxan;
trans-5-Butyl-trans-2'-(4-chlor-3,5-difluor-phenethyl)-2,5'-bi-1,3-dioxan.
trans-2-(4-Trifluormethoxy-phenethyl)-5-[2-(trans-5-propyl-1,3-dioxan-2-yl)-ethyl]-1,3-dioxan;
trans-2-(4-Trifluormethoxy-phenethyl)-5- [2-(trans-5-butyl-1,3-dioxan-2-yl)-ethyl]-1,3-dioxan, Smp. (C-S_{B}) 103,4°C, Klp. (S_{B}-I) 137,3°C;
trans-2-(4-Trifluormethoxy-phenethyl)-5-[2-(trans-5-pentyl-1,3-dioxan-2-yl)-ethyl]-1,3-dioxan;
trans-2-(3,4-Difluor-phenethyl)-5-[2-(trans-5-propyl-1,3-dioxan-2-yl)-ethyl]-1,3-dioxan;
trans-2-(3,4-Difluor-phenethyl)-5-[2-(trans-5-butyl-1,3-dioxan-2-yl)-ethyl]-1,3-dioxan;
trans-2-(3,4-Difluor-phenethyl)-5-[2-(trans-5-pentyl-1,3-dioxan-2-yl)-ethyl]-1,3-dioxan;
(E)-trans-2-(3,4-Difluor-phenethyl)-5-[2-(trans-5-propenyl-1,3-dioxan-2-yl)-ethyl]-1,3-dioxan;
(E)-trans-2-(3,4-Difluor-phenethyl)-5-[2-(trans-5-but-1-enyl-1,3-dioxan-2-yl)-ethyl]-1,3-dioxan, Smp. (C-S_{B}) 99,6°C, Klp. (S_{B}-I) 105,7°C;
trans-2-(4-Difluormethoxy-3-fluor-phenethyl)-5-[2-(trans-5-propyl-1,3-dioxan-2-yl)-ethyl]-1,3-dioxan;
trans-2-(4-Difluormethoxy-3-fluor-phenethyl)-5-[2-(trans-5-butyl-1,3-dioxan-2-yl)-ethyl]-1,3-dioxan;
trans-2-(4-Difluormethoxy-3-fluor-phenethyl)-5-[2-(trans-5-pentyl-1,3-dioxan-2-yl)-ethyl]-1,3-dioxan;
trans-2-(3,4,5-Trifluor-phenethyl)-5-[2-(trans-5-propyl-1,3-dioxan-2-yl)-ethyl] 1,3-dioxan;
trans-2-(3,4,5-Trifluor-phenethyl)-5-[2-(trans-5-butyl-1,3-dioxan-2-yl)-ethyl] 1,3-dioxan;
trans-2-(3,4,5-Trifluor-phenethyl)-5-[2-(trans-5-pentyl-1,3-dioxan-2-yl)-ethyl] 1,3-dioxan;
trans-2-(4-Difluormethoxy-3,5-difluor-phenethyl)-5-[2-(trans-5-propyl-1,3-dioxan-2-yl)-ethyl] 1,3-dioxan;
trans-2-(4-Difluormethoxy-3,5-difluor-phenethyl)-5-[2-(trans-5-butyl-1,3-dioxan-2-yl)-ethyl]1,3-dioxan;
trans-2-(4-Difluormethoxy-3,5-difluor-phenethyl)-5-[2-(trans-5-pentyl-1,3-dioxan-2-yl)-ethyl] 1,3-dioxan;

### Beispiel 5

Zur Untersuchung der Eigenschaften der Verbindungen der Formel I in Gemischen wurden binäre Mischungen (BM) mit 4-(trans-4-Pentylcyclohexyl)-benzonitril hergestellt. Die Schwellenspannung (V₁₀) und die Ansprechzeiten (tₒₙ und t_{off}) wurden in einer TN-Zelle (low bias tilt) mit 8 µm Plattenabstand bei 22°C gemessen; als Betriebsspannung wurde der 2,5-fache Wert der Schwellenspannung gewählt. Die entsprechenden Daten für 4-(trans-4-Pentylcyclohexyl)-benzonitril betragen: Klp. (N-I) 54,6°C, V₁₀ = 1,62V, tₒₙ = 22 ms, t_{off} = 42 ms, Δn = 0,120.

| BM-1 | |
|---|---|
| 90 Gew.% | 4-(trans-4-Pentylcyclohexyl)-benzonitril |
| 10 Gew.% | trans-2-Propyl-5-[2-(trans-5-propyl-1,3-dioxan-2-yl)-ethyl]-1,3-dioxan |

Klp. (N-I) 46,2°C, V₁₀ = 1,43V, tₒₙ = 30 ms, t_{off} = 45 ms, Δn = 0,105.

| BM-2 | |
|---|---|
| 80 Gew.% | 4-(trans-4-Pentylcyclohexyl)-benzonitril |
| 20 Gew.% | trans-2-Propyl-5-[2-(trans-5-propyl-1,3-dioxan-2-yl)-ethyl]-1,3-dioxan |

Klp. (N-I) 38,2°C, V₁₀ = 1,29V, tₒₙ = 36 ms, t_{off} = 63 ms, Δn = 0,096.

| BM-3 | |
|---|---|
| 90 Gew.% | 4-(trans-4-Pentylcyclohexyl)-benzonitril |
| 10 Gew.% | trans-2-Propyl-5-[2-(trans-5-pentyl-1,3-dioxan-2-yl)-ethyl]-1,3-dioxan |

Klp. (N-I) 48,0°C, V₁₀ = 1,38V, tₒₙ = 34 ms, t_{off} = 51 ms, Δn = 0,104.

| BM-4 | |
|---|---|
| 80 Gew.% | 4-(trans-4-Pentylcyclohexyl)-benzonitril |
| 20 Gew.% | trans-2-Propyl-5-[2-(trans-5-pentyl-1,3-dioxan-2-yl)-ethyl]-1,3-dioxan |

Klp. (N-I) 42,2°C, V₁₀ = 1,31V, tₒₙ = 43 ms, t_{off} = 65 ms, Δn = 0,098.

| BM-5 | |
|---|---|
| 90 Gew.% | 4-(trans-4-Pentylcyclohexyl)-benzonitril |
| 10 Gew.% | (E)-trans-2-Propyl-5-[2-(trans-5-propenyl-1,3-dioxan-2-yl)-ethyl]-1,3-dioxan |

Klp. (N-I) 46,4°C, V₁₀ = 1,46V, tₒₙ = 30 ms, t_{off} = 51 ms.

| BM-6 | |
|---|---|
| 80 Gew.% | 4-(trans-4-Pentylcyclohexyl)-benzonitril |
| 20 Gew.% | (E)-trans-2-Propyl-5-[2-(trans-5-propenyl-1,3-dioxan-2-yl)-ethyl]-1,3-dioxan |

Klp. (N-I) 39,4°C, V₁₀ = 1,32V, tₒₙ = 37 ms, t_{off} = 63 ms.

| BM-7 | |
|---|---|
| 90 Gew.% | 4-(trans-4-Pentylcyclohexyl)-benzonitril |
| 10 Gew.% | trans-2-(trans-4-Butylcyclohexyl)-5-[2-(trans-5-pentyl-1,3-dioxan-2-yl)-ethyl]-1,3-dioxan |

Klp. (N-I) 58,6°C, V₁₀ = 1,62V, tₒₙ = 29 ms, t_{off} = 49 ms.

| BM-8 | |
|---|---|
| 90 Gew.% | 4-(trans-4-Pentylcyclohexyl)-benzonitril |
| 10 Gew.% | trans-2-(3,4-Difluorphenyl)-5-[2-(trans-5-propyl-1,3-dioxan-2-yl)-ethyl]-1,3-dioxan |

Klp. (N-I) 51,3°C, V₁₀ = 1,46V, tₒₙ = 30 ms, t_{off} = 49 ms, Δn = 0,116.

| BM-9 | |
|---|---|
| 80 Gew.% | 4-(trans-4-Pentylcyclohexyl)-benzonitril |
| 20 Gew.% | trans-2-(3,4-Difluorphenyl)-5-[2-(trans-5-propyl-1,3-dioxan-2-yl)-ethyl]-1,3-dioxan |

Klp. (N-I) 49,0°C, V₁₀ = 1,28V, tₒₙ = 40 ms, t_{off} = 67 ms, Δn = 0,114.

| BM-10 | |
|---|---|
| 90 Gew.% | 4-(trans-4-Pentylcyclohexyl)-benzonitril |
| 10 Gew.% | (E)-trans-2-(3,4-Difluorphenyl)-5-[2-(trans-5-propenyl-1,3-dioxan-2-yl)-ethyl]-1,3-dioxan |

Klp. (N-I) 52,1°C, V₁₀ = 1,46V, tₒₙ = 30 ms, t_{off} = 51 ms, Δn = 0,117.

| BM-11 | |
|---|---|
| 90 Gew.% | 4-(trans-4-Pentylcyclohexyl)-benzonitril |
| 10 Gew.% | trans-2-(3,4,5-Trifluorphenyl)-5-[2-(trans-5-propyl-1,3-dioxan-2-yl)-ethyl]-1,3-dioxan |

Klp. (N-I) 49,2°C, V₁₀ = 1,37V, tₒₙ = 32 ms, t_{off} = 52 ms, Δn = 0,113.

| BM-12 | |
|---|---|
| 90 Gew.% | 4-(trans-4-Pentylcyclohexyl)-benzonitril |
| 10 Gew.% | (E)-trans-2-(3,4,5-Trifluorphenyl)-5-[2-(trans-5-propenyl-1,3-dioxan-2-yl)-ethyl]-1,3-dioxan |

Klp. (N-I) 50,0°C, V₁₀ = 1,38V, tₒₙ = 31 ms, t_{off} = 53 ms, Δn = 0,115.

## Patentansprüche

1. Verbindungen der allgemeinen Formel 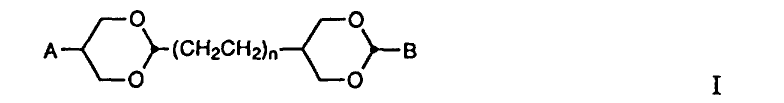 worin
n = 0,1 oder 2;
A und B unabhängig voneinander -R¹, 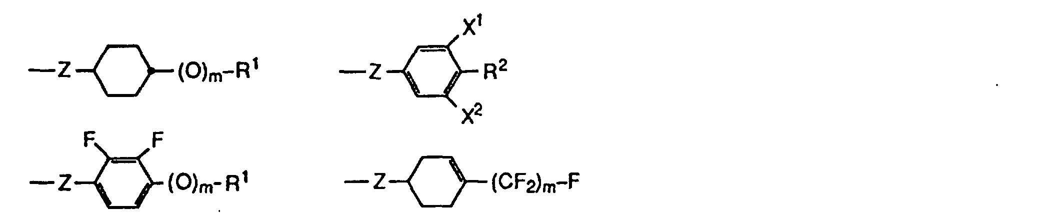 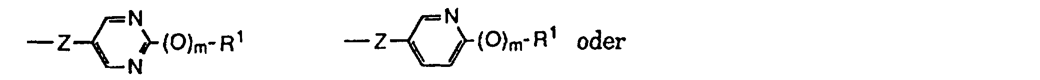 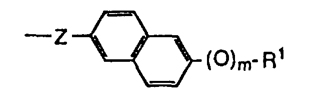 bedeuten
worin
Z eine Einfachbindung, -CH₂CH₂- oder -(CH₂)₄-;
X¹ und X² unabhängig voneinander Wasserstoff oder Fluor;
m 0 oder 1;
R¹ Alkyl, Alkenyl oder Alkoxyalkyl mit höchstens 12 Kohlenstoffatomen, welche gegebenenfalls einfach oder mehrfach mit Fluor und/oder Chlor substituiert sein können; und
R² R¹, -O-R¹, Fluor, Chlor, Cyano oder Isothiocyanato bedeuten.

2. Verbindungen gemäss Anspruch 1 der Formeln 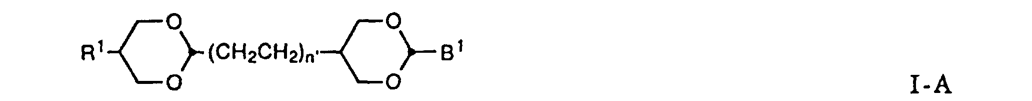 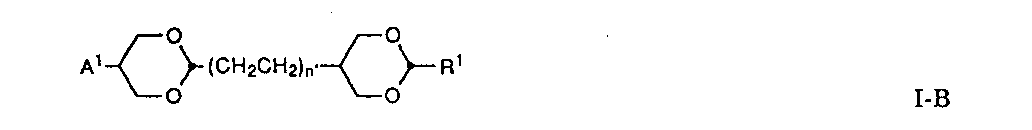 worin
R¹ Alkyl, Alkenyl oder Alkoxyalkyl mit höchstens 12 Kohlenstoffatomen, welche gegebenenfalls einfach oder mehrmals mit Fluor und/oder Chlor substituiert sein können;
A¹ Alkyl, Alkenyl, Alkoxyalkyl oder eine Gruppe der Formel 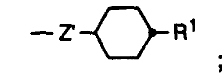 und
B¹ Alkyl, Alkenyl, Alkoxyalkyl oder eine Gruppe der Formel 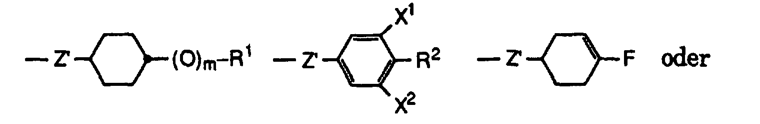 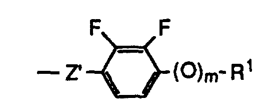 bedeuten; worin
n' 0 oder 1;
m 0 oder 1;
Z' eine Einfachbindung oder -CH₂CH₂-; und
R² R¹, -O-R¹, Fluor, Chlor, Cyano oder Isothiocyanato bedeuten.

3. Verbindungen nach Anspruch 2 der Formeln 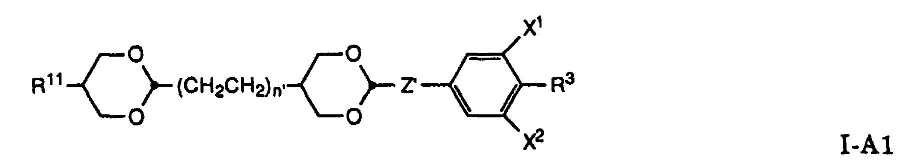 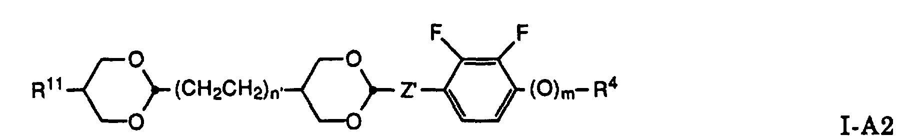 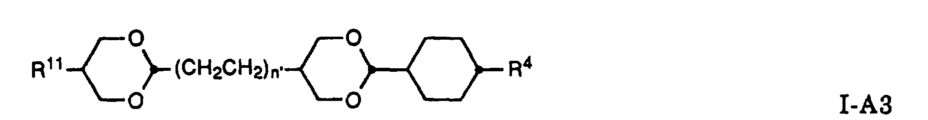 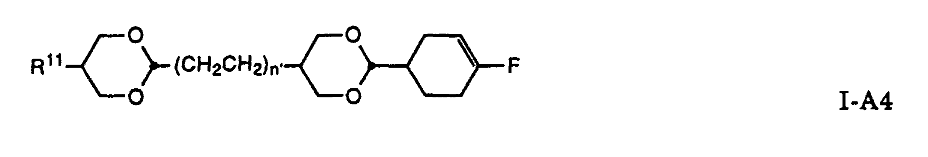 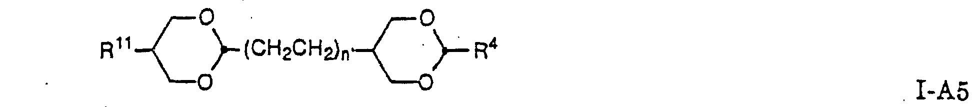 worin
R¹¹ Alkyl oder Alkenyl mit höchstens 7 Kohlenstoffatomen;
n' 0 oder 1;
m 0 oder 1;
X¹ und X² unabhängig voneinander Wasserstoff oder Fluor;
Z' eine Einfachbindung oder -CH₂CH₂-;
R³ Alkosy, Difluormethoxy, Trifluormethoxy, Chlordifluormethoxy, 2,2,2-Trifluorethyloxy, Fluor, Chlor oder Cyano; und
R⁴ Alkyl, Alkosyalkyl oder Alkenyl mit höchstens 7 Kohlenstoffatomen bedeuten.

4. Verbindungen nach Anspruch 2 der Formeln 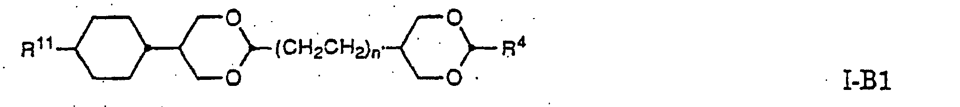 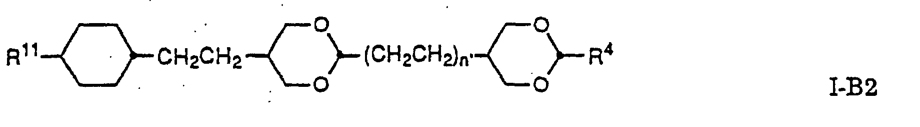 worin
R¹¹ Alkyl oder Alkenyl mit höchstens 7 Kohlenstoffatomen;
n' 0 oder 1; und
R⁴ Alkyl, Alkoxyalkyl oder Alkenyl mit höchstens 7 Kohlenstoffatomen bedeuten.

5. Verbindungen der Formel I-A5 nach Anspruch 3, **dadurch gekennzeichnet, dass** n' = 1 ist.

6. Die Verbindungen nach Anspruch-5
trans-2-Ethyl-5-[2-(trans-5-propyl-1,3-dioxan-2-yl)-ethyl]-1,3-dioxan,
trans-2-Ethy]-5-[2-(trans-5-pentyl-1,3-dioxan-2-yl)-ethyl]-1,3-dioxan,
trans-2-Propyl-5-[2-(trans-5-propyl-1,3-dioxan-2-yl)-ethyl]-1,3-dioxan
trans-2-Propyl-5-[2-(trans-5-butyl-1,3-dioxan-2-yl)-ethyl]-1,3-dioxan,
trans-2-Propyl-5-[2-(trans-5-pentyl-1,3-dioxan-2-yl)-ethyl]-1,3-dioxan
(E)-trans-2-Ethyl-5-[2-(trans-5-propenyl-1,3-dioxan-2-yl)-ethyl]-1,3-dioxan,
(E)-trans-2-Propyl-5-[2-(trans-5-propenyl-1,3-dioxan-2-yl)-ethyl]-1,3-dioxan und
(E)-trans-2-Propyl-5-[2-(trans-5-but-1-enyl-1,3-dioxan-2-yl)-ethyl]-1,3-dioxan.

7. Verbindungen der Formel I-A5 nach Anspruch-3, **dadurch gekennzeichnet, dass** n' = 0 ist.

8. Die Verbindungen nach Anspruch 7
trans-2-Butyl-5-(trans-5-propyl-1,3-dioxan-2-yl)-1,3-dioxan,
trans-2-Butyl-5-(trans-5-butyl-1,3-dioxan-2-yl)-1,3-dioxan,
trans-2-Butyl-5-(trans-5-pentyl-1,3-dioxan-2-yl)-1,3-dioxan,
(E)-trans-2-Butyl-5-(trans-5-propenyl-1,3-dioxan-2-yl)-1,3-dioxan,
(E)-trans-2-Butyl-5-(trans-5-but-1-enyl-1,3-dioxan-2-yl)-1,3-dioxan,
trans-5-Butyl-trans-2'-propyl-2,5'-bi-1,3-dioxan,
trans,trans-5,2'-Dipropyl-2,5'-bi-1,3-dioxan und
trans,trans-5,2'-Dipentyl-2,5'-bi-1,3-dioxan.

9. Verbindungen der Formel I-A3 nach Anspruch 3, **dadurch gekennzeichnet, dass** n' = 1 ist.

10. Die Verbindungen nach Anspruch 9
trans-2-(trans-4-Butylcyclohexyl)-5-[2-(trans-5-propyl-1,3-dioxan-2-yl)-ethyl]-1,3-dioxan,
trans-2-(trans-4-Butylcyclohexyl)-5-[2-(trans-5-butyl-1,3-dioxan-2-yl)-ethyl]-1,3-dioxan,
trans-2-(trans-4-Butylcyclohexyl)-5-[2-(trans-5-pentyl-1,3-dioxan-2-yl)-ethyl]-1,3-dioxan
(E)-trans-2-(trans-4-Butylcyclohexyl)-5-[2-(trans-5-propenyl-1,3-dioxan-2-yl)-ethyl]-1,3-dioxan und
(E)-trans-2-(trans-4-Butylcyclohexyl)-5-[2-(trans-5-but-1-enyl-1,3-dioxan-2-yl)-ethyl]-1,3-dioxan.

11. Die Verbindungen der Formel I-A1 nach Anspruch-3, **dadurch gekennzeichnet, dass** n' = 1 ist und Z' eine Einfachbindung bedeutet.

12. Die Verbindungen nach Anspruch 11,
trans-2-(3,4-Difluorphenyl)-5-[2-(trans-5-propyl-1,3-dioxan-2-yl)-ethyl]-1,3-dioxan
trans-2-(3,4-Difluorphenyl)-5-[2-(trans-5-butyl-1,3-dioxan-2-yl)-ethyl]-1,3-dioxan,
(E)-trans-2-(3,4-Difluorphenyl)-5-[2-(trans-5-propenyl-1,3-dioxan-2-yl)-ethyl]-1,3-dioxan
trans-2-(3,4,5-Trifluorphenyl)-5-[2-(trans-5-propyl-1,3-dioxan-2-yl)-ethyl]-1,3-dioxan
(E)-trans-2-(3,4,5-Trifluorphenyl)-5-[2-(trans-5-propenyl-1,3-dioxan-2-yl)-ethyl]-1,3-dioxan
trans-2-(4-Trifluormethyl-phenyl)-5-[2-(trans-5-propyl-1,3-dioxan-2-yl)-ethyl] 1,3-dioxan,
trans-2-(4-Trifluormethyl-phenyl)-5-[2-(trans-5-butyl-1,3-dioxan-2-yl)-ethyl]-1,3-dioxan und
(E)-trans-2-(4-Trifluormethyl-phenyl)-5-[2-(trans-5-propenyl-1,3-dioxan-2-yl)-ethyl]-1,3-dioxan.

13. Verbindungen der Formel I-A1 nach Anspruch 3, **dadurch gekennzeichnet, dass** n' = 1 ist und Z' -CH₂CH₂- bedeutet.

14. Die Verbindungen nach Anspruch 13
trans-2-(4-Trifluormethoxy-phenethyl)-5-[2-(trans-5-butyl-1,3-dioxan-2-yl)-ethyl]-1,3-dioxan und
(E)-trans-2-(3,4-Difluor-phenethyl)-5-[2-(trans-5-but-1-enyl-1,3-dioxan-2-yl)-ethyl]-1,3-dioxan.

15. Die Verbindungen der Formel I-A1 nach Anspruch 3, **dadurch gekennzeichnet, dass** n' = 0 ist und Z' eine Einfachbindung bedeutet.

16. Die Verbindungen nach Anspruch 15
trans-5-Propyl-trans-2'-(4-trifluormethyl-phenyl)-2,5'-bi-1,3-dioxan,
trans-5-Butyl-trans-2'-(4-trifluormethyl-phenyl)-2,5'-bi-1,3-dioxan,
trans-5-Pentyl-trans-2'-(4-trifluormethyl-phenyl)-2,5'-bi-1,3-dioxan,
trans-5-Propyl-trans-2'-(4-trifluormethoxy-phenyl)-2,5'-bi-1,3-dioxan,
trans-5-Butyl-trans-2'-(4-trifluormethoxy-phenyl)-2,5'-bi-1,3-dioxan,
trans-5-Pentyl-trans-2'-(4-trifluormethoxy-phenyl)-2,5'-bi-1,3-dioxan,
trans-5-Propyl-trans-2'-(3,4-difluor-phenyl)-2,5'-bi-1,3-dioxan,
trans-5-Butyl-trans-2'-(3,4-difluor-phenyl)-2,5'-bi-1,3-dioxan,
trans-5-Pentyl-trans-2'-(3,4-difluor-phenyl)-2,5'-bi-1,3-dioxan,
trans-5-Propyl-trans-2'-(3,4,5-trifluor-phenyl)-2,5'-bi-1,3-dioxan,
trans-5-Butyl-trans-2'-(3,4,5-trifluor-phenyl)-2,5'-bi-1,3-dioxan und
trans-5-Pentyl-trans-2'-(3,4,5-trifluor-phenyl)-2,5'-bi-1,3-dioxan.

17. Die Verbindungen der Formel I-A1 nach Anspruch 3, **dadurch gekennzeichnet, dass** n' = 0 ist und Z' -CH₂CH₂- bedeutet.

18. Die Verbindungen nach Anspruch 17
trans-2'-(3,4-Difluor-phenethyl)-trans-5-butyl-2,5'-bi-1,3-dioxan,
trans-5-Butyl-trans-2'-(4-trifluormethyl-phenethyl)-2,5'-bi-1,3-dioxan,
trans-5-Butyl-trans-2'-(3,4-difluor-phenethyl)-2,5'-bi-1,3-dioxan und
trans-5-Pentyl-trans-2'-(3,4-difluor-phenethyl)-2,5'-bi-1,3-dioxan.

19. Flüssigkristallines Gemisch mit mindestens 2 Komponenten, **dadurch gekennzeichnet, dass** mindestens eine Komponente eine Verbindung der in Anspruch 1 definierten Formel I ist.

20. Flüssigkristallines Gemisch nach Anspruch 19, **dadurch gekennzeichnet, dass** der Anteil an Verbindungen der Formel I 1-30 Gew.-% beträgt.

21. Verwendung der Verbindungen der in Anspruch 1 definierten Formel I für elektro-optische Vorrichtungen.

22. Verwendung von flüssigkristallinen Mischungen nach einem der Ansprüche 19 und 20 für elektro-optische Vorrichtungen.

## Claims

1. Compounds of the general formula 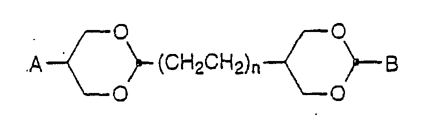 wherein
n is 0, 1 or 2;
A and B each independently signify
-R¹. 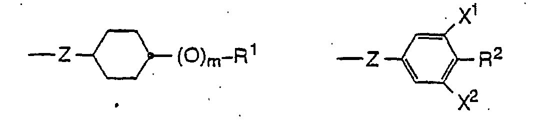 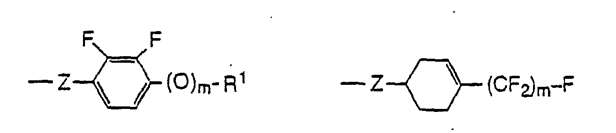 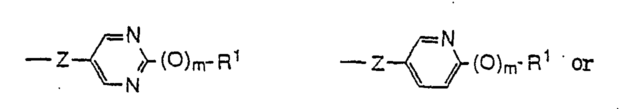 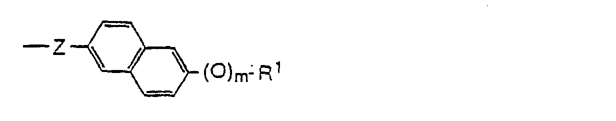 in which
Z signifies a single bond -CH₂CH₂- or -(CH₂)₄-;
X¹ and X² each independently signify hydrogen or fluorine;
m signifies 0 or 1;
R¹ signifies alkyl, alkenyl or alkoxyalkyl with a maximum of 12 carbon atoms, which can be optionally mono- or multiply-substituted with fluorine and/or chlorine; and
R² signifies R¹, -O-R¹, fluorine, chlorine, cyano or isothiocyanato.

2. Compounds according to claim 1 of the formulae 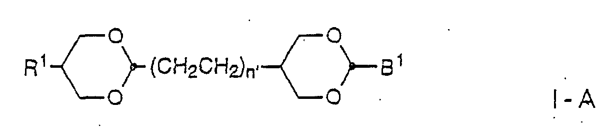 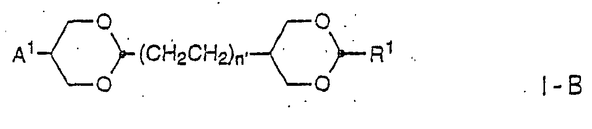 wherein
R¹ signifies alkyl, alkenyl or alkoxyalkyl with a maximum of 12 carbon atoms which can be optionally mono- or multiply-substituted with fluorine and/or chlorine;
A¹ signifies alkyl, alkenyl, alkoxyalkyl or a group of the formula 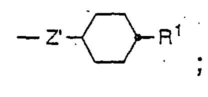 and
B¹ signifies alkyl, alkenyl, alkoxyalkyl or a group of the formula 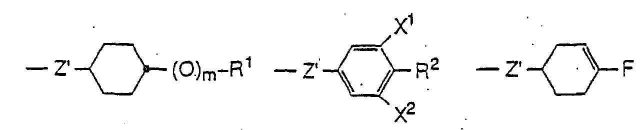 or 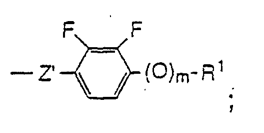 wherein
n' signifies 0 or 1;
m signifies 0 or 1;
Z' signifies a single bond or -CH₂CH₂-; and
R² signifies R¹, -0-R¹ fluorine, chlorine, cyano or isothiocyanato.

3. Compounds according to claim 2 of the formulae 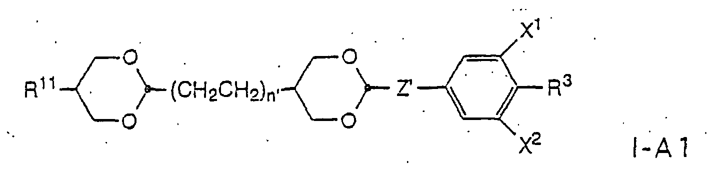 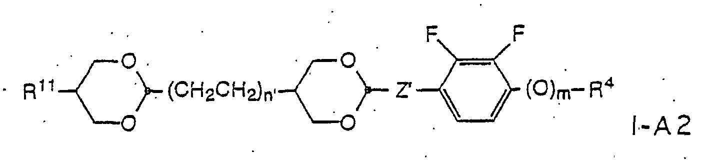 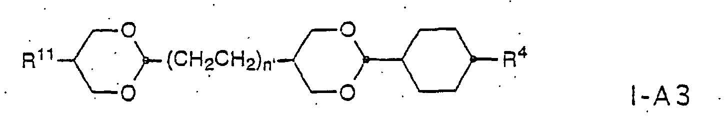 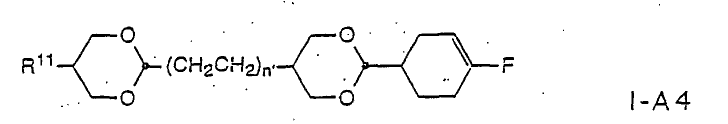 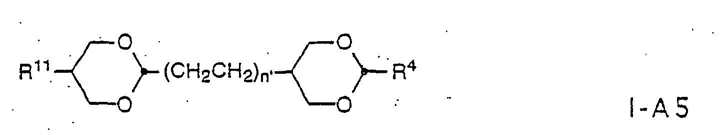 wherein
R¹¹ signifies alkyl or alkenyl with a maximum of 7 carbon. atoms;
n' signifies 0 or 1;
m signifies 0 or 1;
X¹ and X² each independently signify hydrogen or fluorine;
Z' signifies a single bond or -CH₂CH₂-;
R³ signifies alkoxy, difluoromethoxy, trifluoromethoxy, chlorodifluoromethoxy, 2,2,2-trifluoroethyloxy, fluorine, chlorine or cyano; and
R⁴ signifies alkyl, alkoxyalkyl or alkenyl with a maximum of 7 carbon atoms.

4. Compounds according to claim 2 of the formulae 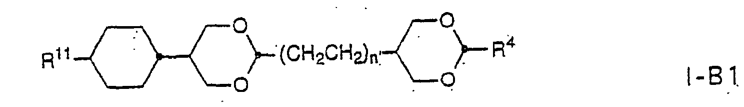 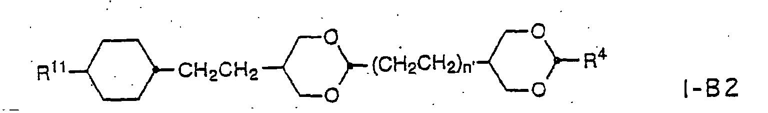 wherein
R¹¹ signifies alkyl or alkenyl with a maximum of 7 carbon atoms;
n' signifies 0 of 1; and
R⁴ signifies alkyl, alkoxyalkyl or alkenyl with a maximum of 7 carbon atoms.

5. Compounds of formula I-A5 according to claim 3, wherein n' = 1.

6. The compounds according to claim 5
trans-2-ethyl-5-[2-(trans-5-propyl-1,3-dioxan-2-yl)-ethyl]-1,3-dioxane,
trans-2-ethyl-5-[2-(trans-5-pentyl-1,3-dioxan-2-yl)-ethyl]-1,3-dioxane,
trans-2-propyl-5-[2-(trans-5-propyl-1,3-dioxan-2-yl)-ethyl]-1,3-dioxane
trans-2-propyl-5-[2-(trans-5-butyl-1,3-dioxan-2-yl)-ethyl]-1,3-dioxane,
trans-2-propyl-5-[2-(trans-5-pentyl-1,3-dioxan-2-yl)-ethyl]-1,3-dioxane
(E)-trans-2-ethyl-5-[2-(trans-5-propenyl-1,3-dioxan-2-yl)-ethyl]-1,3-dioxane,
(E)-trans-2-propyl-5-[2-(trans-5-propenyl-1,3-dioxan-2-yl)-ethyl]-1,3-dioxane and
(E)-trans-2-propyl-5-[2-(trans-5-but-1-enyl-1,3-dioxan-2-yl)-ethyl]-1,3-dioxane.

7. Compounds of formula I-A5 according to claim 3 wherein n' = 0

8. The compounds according to claim 7
trans-2-butyl-5-(trans-5-propyl-1,3-dioxan-2-yl)-1,3-dioxane,
trans-2-butyl-5-(trans-5-butyl-1,3-dioxan-2-yl)-1,3-dioxane,
trans-2-butyl-5-(trans-5-pentyl-1,3-dioxan-2-yl)-1,3-dioxane,
(E)-trans-2-butyl-5-(trans-5-propenyl-1,3-dioxan-2-yl)-1,3-dioxane,
(E)-trans-2-butyl-5-(trans-5-but-1-enyl-1,3-dioxan-2-yl)-1,3-dioxane,
trans-5-butyl-trans-2'-propyl-2,5'-bi-1,3-dioxane,
trans,trans-5,2'-dipropyl-2,5'-bi-1,3-dioxane and
trans,trans-5,2'-dipentyl-2,5'-bi-1,3-dioxane.

9. Compounds of formula I-A3 according to claim 3, wherein n' = 1

10. The compounds according to claim 9
trans-2-(trans-4-butylcyclohexyl)-5-[2-(trans-5-propyl-1,3-dioxan-2-yl)-ethyl]-1,3-dioxane,
tran-2-(trans-4-butylcyclohexyl)-5-[2-(trans-5-butyl-1,3-dioxan-2-yl)-ethyl]-1,3-dioxane,
trans-2-(trans-4-butylcyclohexyl)-5-[2-(trans-5-pentyl-1,3-dioxan-2-yl)-ethyl]-1,3-dioxane
(E)-trans-2-(trans-4-butylcyclohexyl)-5-[2-(trans-5-propenyl-1,3-dioxan-2-yl)-ethyl]-1,3-dioxane and
(E)-trans-2-(trans-4-butylcyclohexyl)-5-[2-(trans-5-but-1-enyl-1,3-dioxan-2-yl)-ethyl]-1,3-dioxane.

11. The compounds of formula I-A1 according to claim 3, wherein n' = 1 and Z' signifies a single bond.

12. The compounds according to claim 11
trans-2-(3,4-difluorophenyl)-5-[2-(trans-5-propyl-1,3-dioxan-2-yl)-ethyl]-1,3-dioxane
trans-2-(3,4-difluorophenyl)-5-[2-(trans-5-butyl-1,3-dioxan-2-yl)-ethyl]-1,3-dioxane,
(E)-trans-2-(3,4-difluorophenyl)-5-[2-(trans-5-propenyl-1,3-dioxan-2-yl)-ethyl]-1,3-dioxane
trans-2-(3,4,5-tnfluorophenyl)-5-[2-(trans-5-propyl-1,3-dioxan-2-yl)-ethyl]-1,3-dioxane
(E)-trans-2-(3,4,5-trifluorophenyl)-5-[2-(trans-5-propenyl-1,3-dioxan-2-yl)-ethyl]-1,3-dioxane
trans-2-(4-trifluoromethyl-phenyl)-5-[2-(trans-5-propyl-1,3-dioxan-2-yl)-ethyl]-1,3-dioxane,
trans-2-(4-trifluoromethyl-phenyl)-5-[2-(trans-5-butyl-1,3-dioxan-2-yl)-ethyl]-1,3-dioxane and
(E)-trans-2-(4-trifluoromethyl-phenyl)-5-[2-(trans-5-propenyl-1,3-dioxan-2-yl)-ethyl]-1,3-dioxane.

13. Compounds of formula I-A1 according to claim 3 wherein n' = 1 and Z' signifies -CH₂CH₂-.

14. The compounds according to claim 13
trans-2-(4-trifluoromethoxy-phenethyl)-5-[2-(trans-5-butyl-1,3-dioxan-2-yl)-ethyl]-1,3-dioxane and
(E)-trans-2-(3,4-difluorophenethyl)-5-[2-(trans-5-but-1-enyl-1,3-dioxan-2-yl)-ethyl]-1,3-dioxane.

15. The compounds of formula I-A1 according to claim 3, wherein n' = 0 and Z' signifies a single bond.

16. The compounds according to claim 15
trans-5-propyl-trans-2'-(4-trifluoromethyl-phenyl)-2,5'-bi-1,3-dioxane
trans-5-butyl-trans-2'(4-trifluoromethyl-phenyl)-2,5'-bi-1,3- . dioxane
trans-5-pentyl-trans-2'-(4-trifluoromethyl-phenyl)-2,5'-bi-1,3-dioxane,
trans-5-propyl-trans-2'-(4-trifluoromethoxy-phenyl)-2,5'-bi-1,3-dioxane,
trans-5-butyl-trans-2'-(4-trifluoromethoxy-phenyl)-2,5'-bi-1,3-dioxane,
trans-5-pentyl-trans-2'-(4-trifluoromethoxy-phenyl)-2,5'-bi-1,3-dioxane,
trans-5-propyl-trans-2'-(3,4-difluoro-phenyl)-2,5'-bi-1,3-dioxane,
trans-5-butyl-trans-2'-(3,4-difluoro-phenyl)-2,5'-bi-1,3-dioxane,
trans-5-pentyl-trans-2'-(3,4-difluoro-phenyl)-2,5'-bi-1,3-dioxane,
trans-5-propyl-trans-2'-(3,4,5-trifluoro-phenyl)-2,5'-bi-1,3-dioxane,
trans-5-butyl-trans-2'-(3,4,5-trifluoro-phenyl)-2,5'-bi-1,3-dioxane and
trans-5-pentyl-trans-2'-(3,4,5-trifluoro-phenyl)-2,5'-bi-1,3-dioxane.

17. The compounds of formula 1-A1 according to claim 3 wherein n' = 0 and Z' signifies -CH₂CH₂-.

18. The compounds according to claim 17
trans-2'-(3,4-difluoro-phenethyl)-trans-5-butyl-2,5'-bi-1,3-dioxane,
trans-5-butyl-trans-2'-(4-trifluoromethyl-phenethyl)-2,5'-bi-1,3-dioxane,
trans-5-butyl-trans-2'-(3,4-difluoro-phenethyl)-2,5'-bi-1,3-dioxane and
trans-5-pentyl-trans-2'-(3,4-difluoro-phenethyl)-2,5'-bi-1,3-dioxane.

19. A liquid crystalline mixture having at least 2 components, wherein at least one component is a compound of formula I defined in claim 1.

20. A liquid crystalline mixture according to claim 19, wherein the content of compounds of formula I is 1-30 wt.%.

21. The use of compounds of formula I defined in claim 1 for electro-optical devices.

22. The use of liquid crystalline mixtures according to either of claims 19 and 20 for electro-optical devices.

## Revendications

1. Composés de formule générale 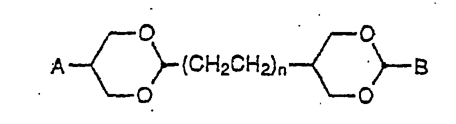 dans laquelle
n = 0, 1 ou 2 ;
A et B représentent indépendamment -R¹, 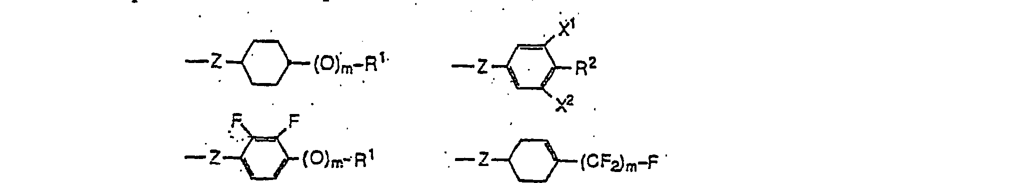 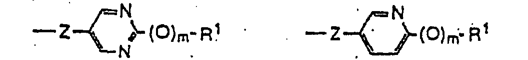 ou 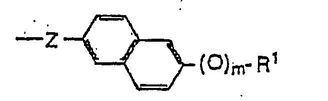 formules dans lesquelles
Z représente une liaison simple, -CH₂-CH₂- ou -(CH₂)₄- ;
X¹ et X² représentent indépendamment un atome d'hydrogène ou de fluor ;
m est égal à 0 ou 1 ;
R¹ représente un groupe alkyle, alcényle ou alcoxyalkyle, comprenant au plus 12 atomes de carbone, qui peuvent être substitués une ou plusieurs fois par des atomes de fluor et/ou de chlore ; et
R² représente R¹, -O-R¹, un atome de fluor ou de chlore, un groupe cyano ou isothiocyanato.

2. Composés selon la revendication 1, ayant les formules 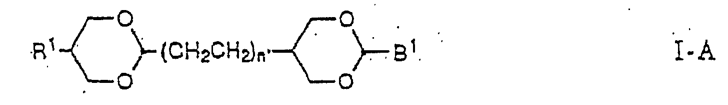 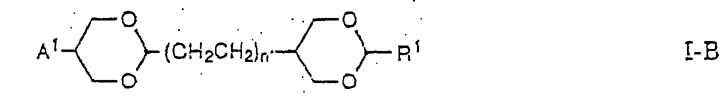 dans lesquelles
R¹ représente un groupe alkyle, alcényle ou alcoxyalkyle, comprenant au plus 12 atomes de carbone, qui peuvent être substitués une ou plusieurs fois par des atomes de fluor et/ou de chlore ; et
A¹ représente un groupe alkyle, alcényle, alcoxyalkyle ou un groupe de formule 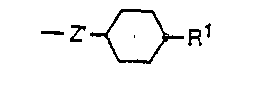 et
B¹ représente un groupe alkyle, alcényle, alcoxyalkyle ou un groupe de formule 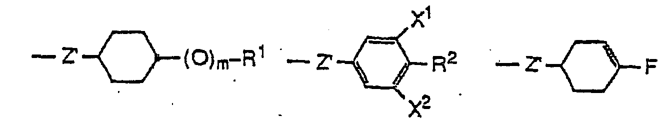 ou 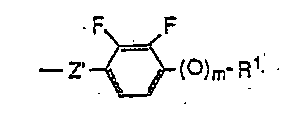 dans lesquelles
n' est égal à 0 ou 1 ;
m est égal à 0 ou 1 ;
Z' représente une liaison simple ou -CH₂-CH₂- ; et
R² représente R¹, -O-R¹, un atome de fluor ou de chlore, un groupe cvano ou isothiocvanato.

3. Composés selon la revendication 2, ayant les formules 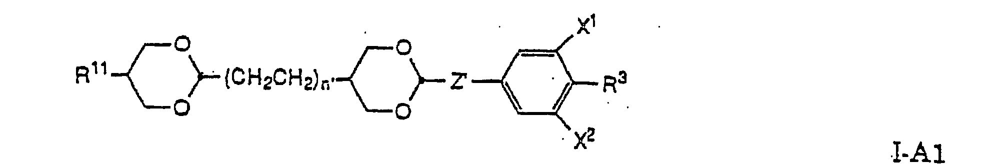 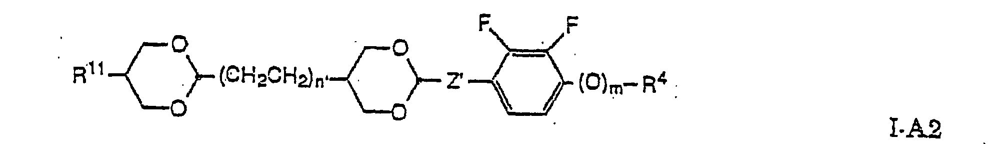 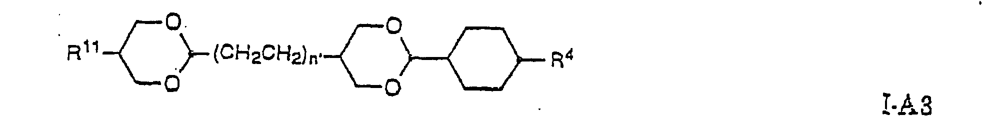 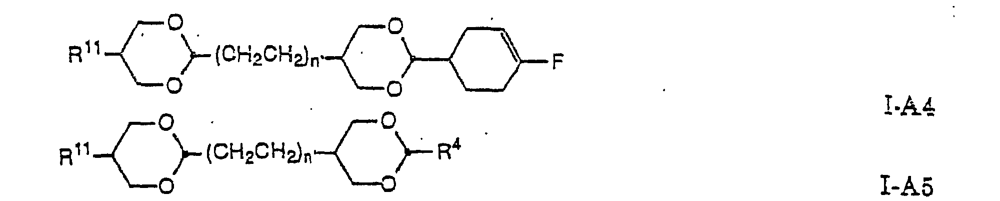 dans lesquelles
R¹¹ représente un groupe alkyle ou alcényle ayant au plus 7 atomes de carbone ;
n' est égal à 0 ou 1 ;
m est égal à 0 ou 1 ;
X¹ et X² représentent indépendamment un atome d'hydrogène ou de fluor :
Z' représente une liaison simple ou -CH₂-CH₂- ;
R³ représente un groupe alcoxy, difluorométhoxy, trifluorométhoxy, chlorodifluorométhoxy, 2,2,2-trifluoroéthyloxy, fluoro, chloro ou cyano ; et
R⁴ représente un groupe alkyle, alcoxyalkyle ou alcényle ayant au plus 7 atomes de carbone.

4. Composés selon la revendication 2, ayant les formules 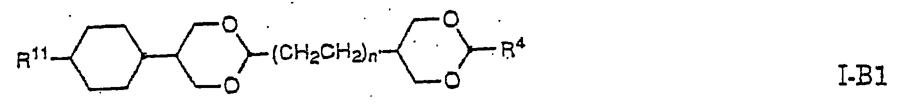 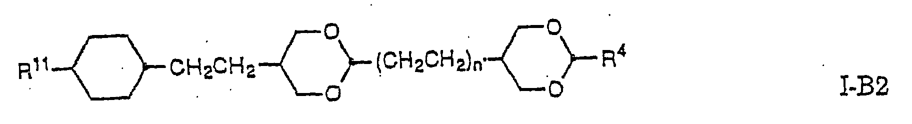 dans lesquelles
R¹¹ représente un groupe alkyle ou alcényle ayant au plus 7 atomes de carbone ;
n' est égal à 0 ou 1 ; et
R⁴ représente un groupe alkyle, alcoxyalkyle ou alcényle avant au plus 7 atomes de carbone.

5. Composés de formule I-A5 selon la revendication 3, **caractérisés par** le fait que n'=1.

6. Composés selon la revendication 5
trans-2-éthyl-5-[2-(trans-5-propyl-1,3-dioxane-2-yl)-éthyl]-1,3-dioxane,
trans-2-éthyl-5-[2-(trans-5-pentyl-1,3-dioxane-2-yl)-éthyl]-1,3-dioxane,
trans-2-propyl-5-[2-(trans-5-propyl-1,3-dioxane-2-yl)-éthyl]-1,3-dioxane,
trans-2-propyl-5-[2-(trans-5-butyl-1,3-dioxane-2-yl)-éthyl]-1,3-dioxane,
trans-2-propyl-5-[2-(trans-5-pentyl-1,3-dioxane-2-yl)-éthyl]-1,3-dioxane, (E)-trans-2-éthyl-5-[2-(trans-5-propényl-1,3-dioxane-2-yl)-éthyl]-1,3-dioxane,
(E)-trans-2-propyl-5-[2-(trans-5-propényl-1,3-dioxane-2-yl)-éthyl]-1,3-dioxane,
et
(E)-trans-2-propyl-5-[2-(trans-5-but-1-ényl-1,3-dioxane-2-yl)-éthyl]-1,3-dioxane.

7. Composés de formule I-A5 selon la revendication 3, **caractérisés par** le fait que n' = 0.

8. Composés selon la revendication 7,
trans-2-butyl-5-(trans-5-propyl-1,3-dioxane-2-yl)-1,3-dioxane,
trans-2-butyl-5-(trans-5-butyl-1,3-dioxane-2-yl)-1,3-dioxane,
trans-2-butyl-5-(trans-5-pentyl-1,3-dioxane-2-yl)-1,3-dioxane,
(E)-trans-2-butyl-5-(trans-5-propényl-1,3-dioxane-2-yl)-1,3-dioxane,
(E)-trans-2-butyl-5-(trans-5-but-1-ényl-1,3-dioxane-2-yl)-1,3-dioxane,
trans-5-butyl-trans-2'-propyl-2,5'-bi-1,3-dioxane,
trans,trans-5,2'-dipropyl-2,5'-bi-1,3-dioxane et
trans,trans-5,2'-dipentyl-2,5'-bi-1,3-dioxane.

9. Composés de formule I-A3 selon la revendication 3, **caractérisés par** le fait que n'= 1.

10. Composés selon la revendication 9,
trans-2-(trans-4-butylcyclohexyl)-5-[2-(trans-5-propyl-1,3-dioxane-2-yl)-éthyl]-1,3-dioxane,
trans-2-(trans-4-butylcyclohexyl)-5-[2-(trans-5-butyl-1,3-dioxane-2-yl)-éthyl]-1,3-dioxane,
trans-2-(trans-4-butylcyclohexyl)-5-[2-(trans-5-pentyl-1,3-dioxane-2-yl)-éthyl]-1,3-dioxane,
(E)-trans-2-(trans-4-butylcyclohexyl)-5-[2-(trans-5-propényl-1,3-dioxane-2-yl)-éthyl]-1,3-dioxane et
(E)-trans-2-(trans-4-butylcyclohexyl)-5-[2-(trans-5-but-1-ényl-1,3-dioxane-2-yl)-éthyl]-1,3-dioxane.

11. Composés de formule I-A1 selon la revendication 3, **caractérisés par** le fait que n' =1 et Z' représente une liaison simple.

12. Composés selon la revendication 11,
trans-2-(3,4-difluorophényl)-5-[2-(trans-5-propyl-1,3-dioxane-2-yl)-éthyl]-1,3-dioxane,
trans-2-(3,4-difluorophényl)-5-[2-(trans-5-butyl-1,3-dioxane-2-yl)-éthyl]-1,3-dioxane,
(E)-trans-2-(3,4-difluorophényl)-5-[2-(trans-5-propényl-1,3-dioxane-2-yl)-éthyl]-1,3-dioxane,
trans-2-(3,4,5-trifluorophényl)-5-[2-(trans-5-propyl-1,3-dioxane-2-yl)-éthyl]-1,3-dioxane,
(E)-trans-2-(3,4,5-trifluorophényl)-5-[2-(trans-5-propényl-1,3-dioxane-2-yl)-éthyl]-1,3-dioxane
trans-2-(4-trifluorométhylphényl)-5-[2-(trans-5-propyl-1,3-dioxane-2-yl)-éthyl]-1,3- dioxane,
trans-2-(4-trifluorométhylphényl)-5-[2-(trans-5-butyl-1,3-dioxane-2-yl)-éthyl]-1,3-dioxane et
(E)-trans-2-(4-trifluorométhylphényl)-5-[2-(trans-5-propényl-1,3-dioxane-2-yl)-éthyl]-1,3-dioxane.

13. Composés de formule I-A1 selon la revendication 3, **caractérisés par** le fait que n'=1 et Z' représente -CH₂CH₂-.

14. Composés selon la revendication 13,
trans-2-(4-trifluorométhoxy-phénéthyl)-5-[2-(trans-5-butyl-1,3-dioxane-2-yl)-éthyl]-1,3-dioxane et
(E)-trans-2-(3,4-difluoro-phénéthyl)-5-[2-(trans-5-but-1-ényl-1,3-dioxane-2-yl)éthyl]-1,3-dioxane.

15. Composés de formule I-A1 selon la revendication 3, **caractérisés par** le fait que n'= O et Z' représente une liaison simple.

16. Composés selon la revendication 15,
trans-5-propyl-trans-2'-(4-trifluorométhylphényl)-2,5'-bi-1,3-dioxane,
trans-5-butyl-trans-2'-(4-trifluorométhylphényl)-2,5'-bi-1,3-dioxane,
trans-5-pentyl-trans-2'-(4-trifluorométhylphényl)-2,5'-bi-1,3-dioxane,
trans-5-propyl-trans-2'-(4-trifluorométhoxyphényl)-2,5'-bi-1,3-dioxane,
trans-5-butyl-trans-2'-(4-trifluorométhoxyphényl)-2,5'-bi-1,3-dioxane,
trans-5-pentyl-trans-2'-(4-trifluorométhoxyphényl)-2,5'-bi-1,3-dioxane
trans-5-propyl-trans-2'-(3,4-difluorophényl)-2,5'-bi-1,3-dioxane,
trans-5-butyl-trans-2'-(3,4-difluorophényl)-2,5'-bi-1,3-dioxane,
trans-5-pentyl-trans-2'-(3,4-difluorophényl)-2,5'-'bi-1,3-dioxane,
trans-5-propyl-trans-2'-(3,4,5-trifluorophényl)-2,5'-bi-1,3-dioxane,
trans-5-butyl-trans-2'-(3,4,5-trifluorophényl)-2,5'-bi-1,3-dioxane et
trans-5-pentyl-trans-2'-(3,4,5-trifluorophényl)-2,5'-bi-1,3-dioxane.

17. Composés de formule I-A1 selon la revendication 3, **caractérisés par** le fait que n'=0 et Z' représente -CH₂CH₂-.

18. Composés selon la revendication 17
trans-2'-(3,4-difluorophénéthyl)-trans-5-butyl-2,5'-bi-1,3-dioxane,
trans-5-butyl-trans-2'-(4-triflurométhyl-phénéthyl)-2,5'-bi-1,3-dioxane
trans-5-butyl-trans-2'-(3,4-difluorophénéthyl)-2,5'-bi-1,3-dioxane et
trans-5-pentyl-trans-2'-(3,4-difluorophénéthyl)-2,5'-bi-1,3-dioxane.

19. Mélange cristallin liquide comprenant au moins 2 composants, **caractérisé par** le fait qu'au moins un des composants est un composé de formule I définie dans la revendication 1.

20. Mélange cristallin liquide selon la revendication 19, **caractérisé par** le fait que la proportion des composés de formule I est de 1-30 % en poids.

21. Utilisation des composés de formule I définie dans la revendication 1, dans des dispositifs électro-optiques.

22. Utilisation des mélanges cristallins liquides selon l'une des revendications 19 et 20 dans des dispositifs électro-optiques.
